# EUROPEAN PATENT SPECIFICATION

(11) **EP 3 146 856 B1**
(45) Date of publication and mention of the grant of the patent: **16.03.2022**
(21) Application number: 16188833.4
(22) Date of filing: 14.09.2016
(51) Int. Cl.: A24F 47/00, A61M 5/178, A61M 5/31

(54) **SYRINGE REFILLER FOR AN ELECTRONIC SMOKING DEVICE**
SPRITZENNACHFÜLLER FÜR EINE ELEKTRONISCHE RAUCHVORRICHTUNG
SERINGUE DE REMPLISSAGE POUR UN DISPOSITIF DE CIGARETTE ÉLECTRONIQUE

(30) Priority: 06.09.2016 EP 16187489
(43) Date of publication of application: 29.03.2017
(73) Proprietor: Fontem Holdings 1 B.V., 1083 HN Amsterdam (NL)
(72) Inventor: BIEL, Stefan, 22761 Hamburg (DE); BEER, Andreas, 85630 Grasbrunn (DE)
(74) Representative: Gulde & Partner

(56) References cited:
- EP-A2- 2 366 418
- US-A- 4 572 210
- US-A1- 2002 165 496
- US-A1- 2014 209 109

## Description

### FIELD OF INVENTION

The present invention relates generally to syringe refillers for electronic smoking devices and in particular electronic cigarettes.

### BACKGROUND OF THE INVENTION

An electronic smoking device, such as an electronic cigarette (e-cigarette), typically has a housing accommodating an electric power source (e.g. a single use or rechargeable battery, electrical plug, or other power source), and an electrically operable atomizer. The atomizer vaporizes or atomizes liquid supplied from a reservoir and provides vaporized or atomized liquid as an aerosol. Control electronics control the activation of the atomizer. In some electronic cigarettes, an airflow sensor is provided within the electronic smoking device, which detects a user puffing on the device (e.g., by sensing an under-pressure or an air flow pattern through the device). The airflow sensor indicates or signals the puff to the control electronics to power up the device and generate vapor. In other e-cigarettes, a switch is used to power up the e-cigarette to generate a puff of vapor.

Most electronic smoking devices are configured to be refilled when the liquid reservoir of the electronic smoking device is emptied. Often, a procedure for the refill of the liquid reservoir of an electronic smoking device comprises a disassembling of the electronic smoking device in order to expose a refill opening of the liquid reservoir of the electronic smoking device. In most cases, a refill bottle is used to refill the liquid reservoir, wherein the refill bottles of the state of the art mostly comprise a simple pipette like attachment or cap which shall allow for liquid to be transported from the refill bottle into the liquid reservoir via the refill opening.

However, such refill bottles with attachments or caps like the aforementioned often cause the liquid to spill during the refill procedure which can be perceived as highly inconvenient for the user of the electronic smoking device. Furthermore, when refilling the liquid reservoir, it is necessary to avoid dripping liquid into the air tube. Liquid that passes down the air tube into the atomizer may flood the atomizer and temporarily stops the device from working. When the device is then operated to clear the misplaced liquid, this often results in leakage as the misplaced liquid finds its way out of the atomizer through the air passage. Clearing the air passage is also often accompanied by a "gurgling" sound and sensation which users find unpleasant. Moreover, difficulties in refilling an electronic smoking device may cause users to miss the reservoir causing their fingers holding the electronic smoking device to come into contact with the liquid for atomization. Further liquid may spill from the reservoir prior to the mouthpiece of the device being re-attached closing the open end of the reservoir. Often liquid for atomization is relatively greasy and is impregnated with flavors which makes coming into contact unpleasant and undesirable as the liquid needs to be washed off and odors from the liquid may be retained on the hands. Furthermore, there is a risk that users may accidentally ingest the liquid if the liquid is not washed off. Furthermore, the refill systems of the state of the art are often difficult to handle and do not allow for a quick and easy refill of an electronic smoking device.

US 4,572,210 discloses a syringe device for obtaining a gas-free blood sample that has a tubular body connected to an hypodermic needle.

US 2002/0165496 A1 discloses a syringe having an air release assembly slidingly clamped to a syringe barrel, the air release assembly having a lower portion with a slider valve insert and an upper portion with a rearward facing chamber.

US 2014/0209109 A1 discloses a pharmaceutical composition and administration apparatus that includes a portable powered vaporizer with a mouthpiece.

### SUMMARY OF THE INVENTION

In accordance with one aspect of the present invention, there is provided a syringe refiller for an electronic smoking device. The syringe refiller comprises a liquid reservoir comprising a first end with a first opening for the reception of a plunger component and a second end with a second opening for a liquid to be pushed out of the liquid reservoir. Furthermore, the syringe refiller comprises a plunger component comprising a plunger attached to a handle, the plunger being configured slidable along an inner wall of the liquid reservoir, wherein the plunger seals the liquid reservoir towards the first opening. The plunger component comprises a hollow body with a first end attached to the plunger and a second end attached to the handle. Further, the plunger comprises a hole for the insertion of an air channel component of an air channelling system. Moreover, the syringe refiller comprises a first syringe needle element that is in connection with the second opening, so that liquid that is pushed out of the second opening via the plunger component flows through the syringe needle element. Furthermore, the syringe refiller comprises an air channeling system which comprises an air channel component inserted into the hole of the plunger, adapted to transport air from outside of the syringe refiller into the hollow body, allowing for air to be sucked into the syringe refiller without interfering with liquid contained therein. Said air channel component comprises a first channel opening and at least one second channel opening, the first channel opening being arranged within the hollow body and the at least one second channel opening being arranged adjacent to the second opening within a sidewall at the second end of the liquid reservoir.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
- Figure 1: is a schematic cross-sectional illustration of an embodiment of an electronic smoking device of an electronic smoking device system;
- Figure 2: is a schematic vertical cross-sectional illustration of a first embodiment of a syringe refiller for an electronic smoking device;
- Figure 3: is a schematic vertical cross-sectional illustration of a second embodiment of a syringe refiller for an electronic smoking device;
- Figure 4: is a schematic vertical cross-sectional illustration of the second embodiment of a syringe refiller in use;
- Figure 5a: is a schematic horizontal cross-sectional illustration of the front part of the second embodiment of a syringe refiller in a locked state;
- Figure 5b: is a schematic horizontal cross-sectional illustration of the front part of the second embodiment of a syringe refiller in an unlocked state;
- Figure 6: is a schematic perspective view of the second embodiment of a syringe refiller in an unlocked state;
- Figure 7: is a schematic perspective view of the second embodiment of a syringe refiller in a locked state;
- Figure 8: shows an embodiment of an electronic smoking device system with an embodiment of an electronic smoking device and the second embodiment of a syringe refiller; and
- Figure 9: shows a refilling of the embodiment of the electronic smoking device of the electronic smoking device system using the second embodiment of a syringe refiller.

### DESCRIPTION OF THE PREFERRED EMBODIMENTS

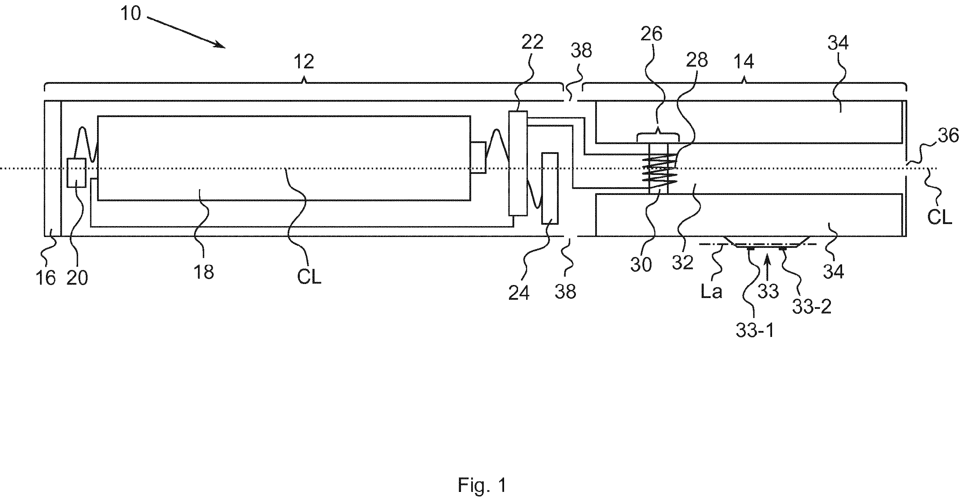

Throughout the following, an electronic smoking device 10 of an electronic smoking device system will be exemplarily described with reference to an e-cigarette. As is shown in Figure 1, an e-cigarette 10 typically has a housing comprising a cylindrical hollow tube having an end cap 16. The cylindrical hollow tube may be a single-piece or a multiple-piece tube. In Figure 1, the cylindrical hollow tube is shown as a two-piece structure having a power supply portion 12 and an atomizer/liquid reservoir portion 14. Together the power supply portion 12 and the atomizer/liquid reservoir portion 14 form a cylindrical tube which can be approximately the same size and shape as a conventional cigarette, typically about 100 mm with a 7.5 mm diameter, although lengths may range from 70 to 150 or 180 mm, and diameters from 5 to 28 mm.

The power supply portion 12 and atomizer/liquid reservoir portion 14 are typically made of metal, e.g. steel or aluminum, or of hardwearing plastic and act together with the end cap 16 to provide a housing to contain the components of the e-cigarette 10. The power supply portion 12 and an atomizer/liquid reservoir portion 14 may be configured to fit together by a friction push-fit, a snap fit, or a bayonet attachment, magnetic fit, or screw threads. The end cap 16 is provided at the front end of the power supply portion 12. The end cap 16 may be made from translucent plastic or other translucent material to allow a light-emitting diode (LED) 20 positioned near the end cap to emit light through the end cap. The end cap can be made of metal or other materials that do not allow light to pass.

An air inlet may be provided in the end cap, at the edge of the inlet next to the cylindrical hollow tube, anywhere along the length of the cylindrical hollow tube, or at the connection of the power supply portion 12 and the atomizer/liquid reservoir portion 14. Figure 1 shows a pair of air inlets 38 provided at the intersection between the power supply portion 12 and the atomizer/liquid reservoir portion 14.

A power supply, preferably a battery 18, an LED 20, control electronics 22 and optionally an airflow sensor 24 are provided within the cylindrical hollow tube power supply portion 12. The battery 18 is electrically connected to the control electronics 22, which are electrically connected to the LED 20 and the airflow sensor 24. In this example, the LED 20 is at the front end of the power supply portion 12, adjacent to the end cap 16 and the control electronics 22 and airflow sensor 24 are provided in the central cavity at the other end of the battery 18 adjacent to the atomizer/liquid reservoir portion 14.

The airflow sensor 24 acts as a puff detector, detecting a user puffing or sucking on the atomizer/liquid reservoir portion 14 of the e-cigarette 10. The airflow sensor 24 can be any suitable sensor for detecting changes in airflow or air pressure, such as a microphone switch including a deformable membrane which is caused to move by variations in air pressure. Alternatively, the sensor may be a Hall element or an electro-mechanical sensor.

The control electronics 22 are also connected to an atomizer 26. In the example shown, the atomizer 26 includes a heating coil 28 which is wrapped around a wick 30 extending across a central passage 32 of the atomizer/liquid reservoir portion 14. The coil 28 may be positioned anywhere in the atomizer 26 and may be transverse or parallel to the liquid reservoir 34. The wick 30 and heating coil 28 do not completely block the central passage 32. Rather an air gap is provided on either side of the heating coil 28 enabling air to flow past the heating coil 28 and the wick 30. The atomizer may alternatively use other forms of heating elements, such as ceramic heaters, or fiber or mesh material heaters. Nonresistance heating elements such as sonic, piezo and jet spray may also be used in the atomizer in place of the heating coil.

The central passage 32 is surrounded by a cylindrical liquid reservoir 34 with the ends of the wick 30 abutting or extending into the liquid reservoir 34. The wick 30 may be a porous material such as a bundle of fiberglass fibers, with liquid in the liquid reservoir 34 drawn by capillary action from the ends of the wick 30 towards the central portion of the wick 30 encircled by the heating coil 28.

The liquid reservoir 34 may alternatively include wadding soaked in liquid which encircles the central passage 32 with the ends of the wick 30 abutting the wadding. In other embodiments, the liquid reservoir 34 may comprise a toroidal cavity arranged to be filled with liquid and with the ends of the wick 30 extending into the toroidal cavity.

An air inhalation port 36 is provided at the back end of the atomizer/liquid reservoir portion 14 remote from the end cap 16. The inhalation port 36 may be formed from the cylindrical hollow tube atomizer/liquid reservoir portion 14 or may be formed in an end cap.

In use, a user sucks on the e-cigarette 10. This causes air to be drawn into the e-cigarette 10 via one or more air inlets, such as air inlets 38, and to be drawn through the central passage 32 towards the air inhalation port 36. The change in air pressure which arises is detected by the airflow sensor 24, which generates an electrical signal that is passed to the control electronics 22. In response to the signal, the control electronics 22 activate the heating coil 28, which causes liquid present in the wick 30 to be vaporized creating an aerosol (which may comprise gaseous and liquid components) within the central passage 32. As the user continues to suck on the e-cigarette 10, this aerosol is drawn through the central passage 32 and inhaled by the user. At the same time, the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol, more liquid is drawn into the wick 30 from the liquid reservoir 34 by capillary action and is thus available to be converted into an aerosol through subsequent activation of the heating coil 28.

Some e-cigarettes are intended to be disposable and the electric power in the battery 18 is intended to be sufficient to vaporize the liquid contained within the liquid reservoir 34, after which the e-cigarette 10 is thrown away. In this embodiment, the battery 18 is rechargeable and the liquid reservoir 34 is refillable. In the cases where the liquid reservoir 34 is a toroidal cavity, this may be achieved by refilling the liquid reservoir 34 via a refill port. In other embodiments the atomizer/liquid reservoir portion 14 of the e-cigarette 10 is detachable from the power supply portion 12 and a new atomizer/liquid reservoir portion 14 can be fitted with a new liquid reservoir 34 thereby replenishing the supply of liquid. In some cases, replacing the liquid reservoir 34 may involve replacement of the heating coil 28 and the wick 30 along with the replacement of the liquid reservoir 34. A replaceable unit comprising the atomizer 26 and the liquid reservoir 34 is called a cartomizer. In this embodiment, the liquid reservoir 34 comprises an outer refill interface 33 comprising two separate openings 33-1, 33-2 adapted for the reception of two different syringe needle elements (not shown) of a syringe refiller (not shown) which will be described further hereinafter. In other embodiments, the liquid reservoir 34 may comprise an outer refill interface 33 comprising only one opening 33-1 that is adapted for the reception of a syringe needle element of a syringe refiller. In this embodiment, the outer refill interface 33 is arranged such that a line La through the two separate openings 33-1, 33-2 is parallel to a longitudinal center line CL of the electronic smoking device 10. However, the interface can also be arranged such that a line through the two separate openings 33-1, 33-2 is perpendicular to a longitudinal center line CL of the electronic smoking device 10 or such that it has another orientation which is different from the one shown in Figure 1.

The new liquid reservoir 34 may be in the form of a cartridge having a central passage 32 through which a user inhales aerosol. In other embodiments, aerosol may flow around the exterior of the cartridge to an air inhalation port 36.

Of course, in addition to the above description of the structure and function of a typical e-cigarette 10, variations also exist. For example, the LED 20 may be omitted. The airflow sensor 24 may be placed adjacent to the end cap 16 rather than in the middle of the e-cigarette. The airflow sensor 24 may be replaced with a switch which enables a user to activate the e-cigarette manually rather than in response to the detection of a change in air flow or air pressure. Different types of atomizers may be used. Thus, for example, the atomizer may have a heating coil in a cavity in the interior of a porous body soaked in liquid. In this design aerosol is generated by evaporating the liquid within the porous body either by activation of the coil heating the porous body or alternatively by the heated air passing over or through the porous body. Alternatively, the atomizer may use a piezoelectric atomizer to create an aerosol either in combination or in the absence of a heater.

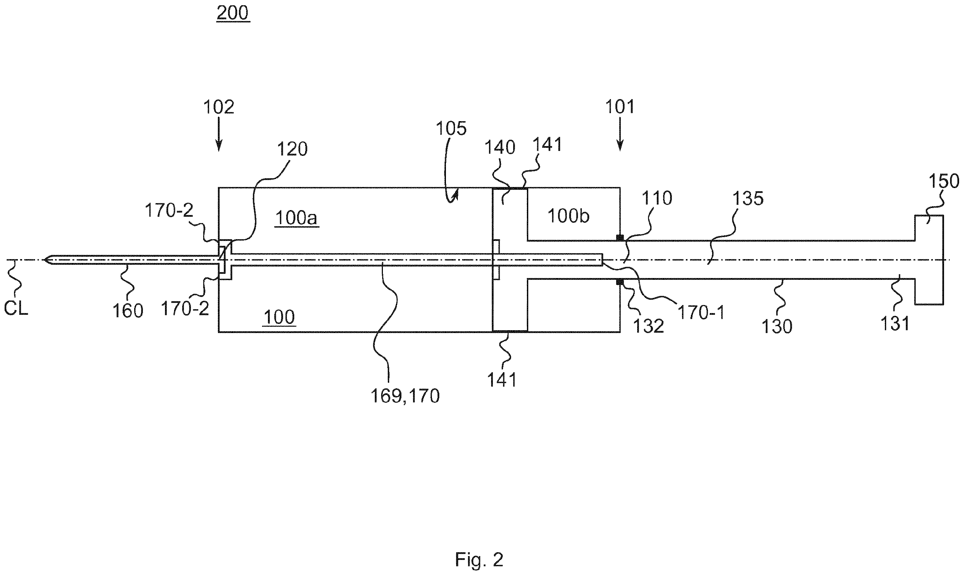

In Figure 2, a schematic vertical cross-sectional illustration of a first embodiment of a syringe refiller 200 for an electronic smoking device is shown. In this first embodiment, the syringe refiller 200 comprises a liquid reservoir 100 that has a first end 101 with a first opening 110 for the reception of a plunger component 130 and a second end 102 with a second opening 120 for a liquid to be pushed out of the liquid reservoir 100. Furthermore, the syringe refiller 200 comprises a plunger component 130 comprising a plunger 140 attached to a handle 150, the plunger 140 being configured slidable along an inner wall 105 of the liquid reservoir 100, wherein the plunger 140 seals the liquid reservoir 100 towards the first opening 110. A first syringe needle element 160 is in connection with the second opening 120, so that liquid which is pushed out of the second opening 120 via the plunger component 130 flows through the syringe needle element 160. Furthermore, the syringe refiller 200 comprises an air channelling system 169 allowing for air to be sucked into the syringe refiller 200 without interfering with liquid contained therein.

An advantage of that may be that an emptied liquid reservoir of an electronic smoking device can easily and quickly be refilled without that a disassembling of the electronic smoking device is necessary. Furthermore, the syringe needle element 160 allows for a precise refilling of the liquid reservoir of an electronic smoking device without that liquid is dripped into the air tube wherein the pressure within the liquid reservoir of an electronic smoking device is equalized. Moreover, the syringe refiller 200, together with an electronic smoking device connected to the syringe refiller 200, represents a closed refill system. This refill system prevents leakage or spilling of liquid as also the air or gas - which can comprise liquid drops - that exits the liquid reservoir of an electronic smoking device during a refill process is recaptured by the air channelling system 169 of the syringe refiller 200.

In this first embodiment, the liquid reservoir 100 substantially has the shape of a cylindrical tube with a predefined volume for the reception of a liquid that is usable for a refill of the liquid reservoir of an electronic smoking device. In this first embodiment, the liquid reservoir 100 of the syringe refiller 200 has a volume that is substantially identical to the volume of the liquid reservoir of a corresponding electronic smoking device refillable by the syringe refiller 200. The liquid reservoir 100 of the syringe refiller 200 comprises a second end 102 with the second opening 120 arranged therein. When liquid is contained within the liquid reservoir 100, it can be expelled via the second opening 120. In more detail, in this first embodiment, the second opening 120 is arranged in a sidewall at the second end 102 of the cylindrical tube-shaped liquid reservoir 100 of the syringe refiller 200. However, also other syringe refillers can be realized with other arrangements of the second opening, for example with the second opening being arranged within a protrusion that is arranged on a sidewall at the second end 102 of the liquid reservoir 100. A first syringe needle element 160 is attached to the second opening 120 of the liquid reservoir 100. The first syringe needle element 160 in this first embodiment is exemplarily realized as a hollow needle that extends along a line that falls together with a center line CL of the liquid reservoir 100 of the syringe refiller 200. Expressed in other words, the first syringe needle element 160 that comes to use in this first embodiment of a syringe refiller 200 is exemplarily equal to the standard syringe needles which also come to use for medical purposes, for example in order to take a blood sample of a human patient. However, also other embodiments of syringe refillers can be realized with other syringe needle elements, for example with shorter and thicker needle elements, especially with syringe needle elements that resemble pins or rods with liquid channels therein.

The plunger component 130 in this first embodiment has a rod shaped shaft 131 that is inserted into the first opening 110 of the liquid reservoir 100 of the syringe refiller 200, the rod shaped shaft 131 having a hollow body 135. Furthermore, in this first embodiment, the first opening 110 comprises an inlay gasket 132 which has the shape of an o-ring and which is inserted into a rim portion of the first opening 110, lining the inner periphery of the first opening 110. The first opening 110 and the inlay gasket 132 are designed such that the rod shaped shaft 131 of the plunger component 130 is moveable relative to the first opening 110. The plunger 140 is attached to the rod shaped shaft 131 and arranged within the liquid reservoir 100 of the syringe refiller 200. It has a shape that fits tightly into the cylindrical tube-shaped liquid reservoir 100, such that the outer walls of the plunger 140 are in direct contact with the inner walls 105 of the cylindrical tube-shaped liquid reservoir 100. In this embodiment, the plunger 140 comprises an outer gasket element 141 that in this first embodiment is arranged at the outer periphery of the plunger 140 and that is in contact with the inner walls 105 of the cylindrical tube-shaped liquid reservoir 100. Expressed in other words, in this first embodiment, the outer walls of the plunger 140 that are in contact with the inner walls 105 of the cylindrical tube-shaped liquid reservoir 100 are exemplarily provided by a part of the surface of the outer gasket element 141. However, also other embodiments of syringe refillers 200 with other plungers 140 can come to use, the plungers 140 being realized with other or without outer gasket elements. Furthermore, it is possible to realize other embodiments of syringe refillers 200 with other plungers 140 that comprise an outer gasket element, wherein the outer walls of the plunger 140 and additionally the surface of the outer gasket element 141 is in contact with the inner walls 105 of the cylindrical tube-shaped liquid reservoir 100.

Via this outer gasket element 141, the plunger 140 seals the liquid reservoir 100 towards the first opening 110. Expressed in other words, the outer gasket element 141 of the plunger 140 and the plunger 140 itself divide the liquid reservoir 100 of the syringe refiller 200 into a first and a second chamber 100a, 100b, wherein the dimensions of the first and the second chambers 100a, 100b are alterable respectively due to the mobility of the plunger 140. The plunger 140 can be pulled and pushed along inside the cylindrical tube-shaped liquid reservoir 100 - which is often also called a barrel - allowing for the syringe refiller 200 to take in and expel a liquid through the second opening 120, which in this embodiment forms an orifice at the second end of the tube-shaped liquid reservoir 100. When the plunger 140 is pulled towards the first end 110 of the liquid reservoir 100, liquid can be sucked into the first chamber 100a which is continuously enlarged while the plunger 140 is pulled towards the first end 110. Furthermore, liquid within the first chamber 100a can be pushed out of the first chamber 100a and be expelled out of the second opening 120 and the first syringe needle element 160 when the plunger 140 is pushed from the first end 110 towards the second end 120 of the liquid reservoir 100 of the syringe refiller 200, thereby decreasing the volume of the first chamber 100a and enlarging the volume of the second chamber 100b. The plunger 140 and the outer gasket element 141 prevent liquid from flowing from the first chamber 100a into the second chamber 100b.

In this first embodiment, the handle 150 has a T-shaped cross-section, allowing for the plunger 140 to be pushed and pulled within the liquid reservoir 100 of the syringe refiller 200. The diameter of the T-shaped handle 150 is larger than the diameter of the rod-shaped shaft 131 in order to prevent the rod-shaped shaft 131 from being fully inserted into the first opening 110. Therefore, liquid can be sucked in and pushed out of the first chamber 100a of the liquid reservoir 100 of the syringe refiller 200 via the generation of an overpressure or an underpressure within the first chamber 100a, moving the plunger component 130 in between the first and the second end 101, 102 of the liquid reservoir 100. However, also other embodiments of syringe refillers with other handles can be realized. For example, a handle attached to the rod-shaped shaft 131 can have the shape of a plate or simply can be realized as a sidewall closing the hollow body 135. Often, the handle comprises a corrugated surface in order to improve the tangibility of the handle 150.

The air channelling system 169 which is allowing for air to be sucked into the syringe refiller 200 without interfering with liquid contained therein in this first embodiment exemplarily comprises an air channel component 170 which is realized as a longitudinal channel that is centrally arranged within the liquid reservoir 100 of the syringe refiller 200. Expressed in other words, in this first embodiment, the air channel component 170 has the shape of an elongated tube that is arranged within the tube-shaped liquid reservoir 100 of the syringe refiller 200. In this first embodiment, the air channel component 170 of the air channelling system 169 pierces through the plunger 140 of the plunger component 130 and extends into the hollow body 135 of the rod shaped shaft 131.

The plunger 140 is also configured slidable along the air channel component 170, wherein the connection between the plunger 140 and the air channel component 170 is tight enough to prevent liquid contained within the liquid reservoir 100 from entering the hollow body 135 via a gap between the plunger 140 and the air channel component 170 or the like. The air channel component 170 has a first channel opening 170-1 and two second channel openings 107-2. The first channel opening 170-1 is arranged within the hollow body 135 of the plunger component 130, notwithstanding the relative position of the hollow body 135 in relation to the air channel component 170. The two second channel openings 107-2 are arranged adjacent to the second opening 120 within the sidewall at the second end 102 of the cylindrical tube-shaped liquid reservoir 100 of the syringe refiller 200. Therefore, the air channel component 170 has a bifurcation close to the second end 102 of the liquid reservoir 100, comprising two tube-shaped channel pieces which extend from the air channel component 170 and together form a U- or a Y-shape. The two tube-shaped channel pieces connect the two second channel openings 170-2 to the air channel component 170. Since the two tube-shaped channel pieces which interconnect the two second channel openings 170-2 to the air channel component 170 each are realized as tubes, they do not cover the second opening 120 within the sidewall on the second end 102 of the tube-shaped liquid reservoir 100. However, also other embodiments of syringe refillers 200 can be realized with other air channelling systems comprising other air channel components. Via the two second channel openings 170-2 the air channelling system 169 of the syringe refiller 200 can be connected to a corresponding outlet opening of an electronic smoking device or in more detail, to a corresponding outlet opening of a liquid reservoir of an electronic smoking device. Therefore, when the liquid reservoir of an electronic smoking device is refilled with liquid via the second opening 120 of the syringe refiller 200, the air that is simultaneously pushed out of the liquid reservoir of the electronic smoking device is led into the hollow body 135 of the syringe refiller 200 via the air channelling system 169. Since the air channelling system 169 and its components are entirely sealed within the liquid reservoir 100, air will not interfere with liquid contained within the reservoir. Expressed in other words, air within the air channelling system 169 will not mix with liquid within the liquid reservoir 100, so that the air within the air channelling system 169 is isolated from the liquid contained within the liquid reservoir 100. Moreover, a further syringe needle element can be connected to the two second channel openings 170-2 respectively, allowing to suck air directly out of an opening provided in a liquid reservoir of an electronic smoking device. Furthermore, also other embodiments of syringe refillers can be carried out, comprising only one or more than two second channel openings 170-2.

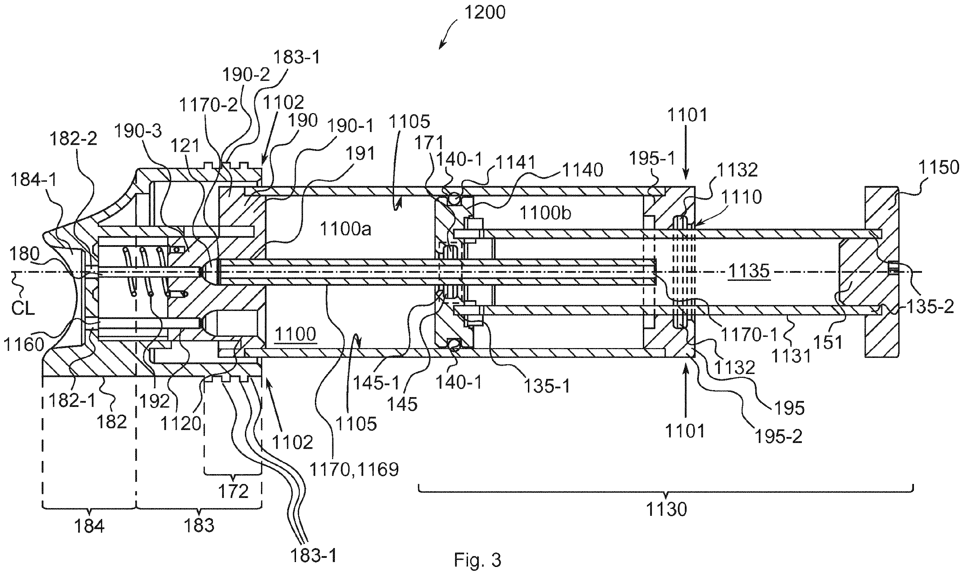

Figure 3 is a schematic vertical cross-sectional illustration of a second embodiment of a syringe refiller 1200 for an electronic smoking device. The syringe refiller 1200 shown in Figure 3 is substantially identical to the syringe refiller 200 shown in Figure 2. Therefore, also in this third embodiment, the syringe refiller 1200 comprises a liquid reservoir 1100 that has the shape of a cylindrical tube. However, also other liquid reservoirs 1100 with other shapes can be realized, for example liquid reservoirs 1100 that have a prismatic shape, a rectangular shape or a cubic shape. The tube-shaped liquid reservoir 1100 comprises a first end 1101 with a first opening 1110 for the reception of a plunger component 1130 and a second end 1102 with a second opening 1120 for a liquid to be pushed out of the liquid reservoir 1100. Moreover, the syringe refiller 1200 also comprises a plunger component 130 comprising a plunger 140 attached to a handle 1150. Also in this second embodiment, the plunger 1140 is configured slidable along an inner wall 1105 of the liquid reservoir 1100, wherein the plunger 1140 seals the liquid reservoir 1100 towards the first opening 1110. A first syringe needle element 1160 is in connection with the second opening 120, so that liquid which is pushed out of the second opening 1120 via the plunger component 1130 flows through the syringe needle element 1160. Moreover, the syringe refiller 1200 comprises an air channelling system 1169 allowing for air to be sucked into the syringe refiller 1200 without interfering with liquid contained therein.

The plunger 1140 in this second embodiment exemplarily has the shape of a piston or of a stamp. The plunger 140 tightly fits into the tube-shaped liquid reservoir 1100 but nevertheless can be pushed and pulled along the inner walls 1105 of the same, being moveable relative to the liquid reservoir 1100. In this second embodiment, the plunger 1140 comprises a peripheral cavity 140-1 that has U-shaped cross-section. An outer gasket element 1141 is arranged within the peripheral cavity 140-1, the outer gasket element 1141 being realized as an o-ring which is in contact with the inner wall 1105 of the tube-shaped liquid reservoir 1100. Thus, the plunger 1140 can be pushed and pulled along the inner wall 1105 of the tube-shaped liquid reservoir 1100, wherein the outer gasket element 1141 is in constant contact with the inner wall 1105 so that the plunger 1140 separates the liquid reservoir 1100 into a first chamber 1100a and a second chamber 1100b. The dimensions of the first chamber 1100a and of the second chamber 1100b depend on the position of the plunger 1140 within the tube-shaped liquid reservoir 1100 respectively. In a first state in which the plunger 1140 is closest to the first end 1101 of the liquid reservoir 1100, the dimension and the volume of the first chamber 1100a is maximized and the dimension and the volume of the second chamber 1100b is minimized. In a second state in which the plunger 1140 is closest to the second end 1102 of the liquid reservoir 1100, the dimension and the volume of the first chamber 1100a is minimized and the dimension and the volume of the second chamber 1100b is maximized.

In this second embodiment, the plunger component 1130 further comprises a hollow body 1135 with a first end 135-1 attached to the plunger 1140 and a second end 135-2 attached to the handle 1150. The plunger 1140 comprises a hole 145 for the insertion of an air channel component 1170 of the air channelling system 1169, adapted to transport air from outside of the syringe refiller 1200 into the hollow body 1135. An advantage of that may be that air, which is pushed out of the liquid reservoir of an electronic smoking device when the same is refilled, can be released into the hollow body 1135 of the syringe refiller 1200. The hollow body 1135 has the shape of a tube with a diameter that is smaller than the diameter of the tube-shaped liquid reservoir 1100. Furthermore, the hollow body 1135 comprises a first end 135-1 and a second end 135-2. At the first end 135-1, the hollow body 1135 protrudes into a circumferential notch arranged within the plunger 1140, the circumferential notch corresponding to the cylindrical walls of the tube-shaped hollow body 1135 of the plunger component 1130. Thus, via the first end 135-1 of the hollow body 1135 of the plunger component 1130, the plunger 1140 is attached to the hollow body 1135 via a press-fit connection. However, the plunger 1140 and the hollow body 1135 can also be connected or attached to each other via another form of connection, for exampled they can be glued together or integrally formed as a single component. The second end 135-2 of the hollow body 1135 is attached to the handle 1150 of the plunger component 1130. In more detail, the handle 1150 has a stopper body 151 which fits into the hollow body 1135 at the second end 135-2 of the same. Furthermore, also at its second end 135-2, the hollow body 1135 protrudes into a circumferential notch arranged within the handle 1150, the circumferential notch corresponding to the cylindrical walls of the tube-shaped hollow body 1135 of the plunger component 1130, representing a fit for the same. Thus, via the second end 135-2 of the hollow body 1135 of the plunger component 1130 engaging with the stopper body 151 and with the circumferential notch of the hollow body 1135, the handle 1150 is attached to the hollow body 1135 via a press-fit connection. However, also other types of connections between the hollow body 1135 and the handle 1150 can be realized. For example, the handle 1150 and the hollow body 1135 can be connected to each other via a screw-fit connection, via a snap-action connection or via a friction push-fit connection. In other embodiments, the handle 1150 can be glued to the hollow body 1135 or the handle 1150 and the hollow body 1135 can be integrally formed as a single component. In this second embodiment, the handle 1150 exemplarily has the shape of a plug with the stopper body 151 being the plugging component.

The hole 145 that is arranged within the plunger 1140 is adapted for the insertion and reception of an air channel component 1170 of the air channelling system 1169. In more detail, the air channelling system 1169 comprises an air channel component 1170 and an internal gasket element 171, wherein the internal gasket element 171 is arranged within the hole 145 of the plunger 1140, providing for an airtight contact between the plunger 1140 and the air channel component 1170 and wherein the plunger 1140 is also configured slidable along the air channel component 1170.

An advantage of that may be that liquid contained within the first chamber 1100a of the liquid reservoir 1100 is not mixed but strictly separated from air that is released into the hollow body 1135 during a pressure equalization within the liquid reservoir of an electronic smoking device that is refilled. Thus, the syringe refiller 1200 and especially the plunger component 1130 can be actuated without that the air channelling system 1169 of the syringe refiller 1200 is affected and without that air entering the syringe refiller 1200 and liquid contained within and/or exiting the syringe refiller 1200 is mixed.

In this second embodiment, the internal gasket element 171 is arranged within the hole 145 of the plunger 1140. The diameter of the hole 145 varies along the width of the plunger 1140, wherein the diameter of the hole 145 is largest in the center area 145-1 of the plunger 1140. In this center area 145-1 of the plunger 1140, the periphery of the hole 145 forms an internal peripheral notch within the plunger 1140 that has an internal diameter which is adjusted to the diameter of the air channel component 1170. The internal peripheral notch within the plunger 1140 has a U-shaped cross-section, wherein the internal gasket element 171 is arranged within this internal peripheral notch within the plunger 1140. Therefore, the shape of the internal gasket element 171 is adjusted to the shape of the air channel component 1170 of the air channelling system 1169. The air channel component 1170 of the air channelling system 1169 is adapted to transport air from outside of the syringe refiller 1200 into the hollow body 1135 of the plunger component 1130. The air channel component 1170 has the shape of a cylindrical rod or tube with a small diameter and in this second embodiment resembles a straw. Furthermore, the air channel component 1170 comprises an internal channel adapted for air to flow through the same. The air channel component 1170 is tightly fitted into the internal gasket element 171 so that liquid from the first chamber 1100a of the liquid reservoir 1100 can not enter the hollow body 1135 of the plunger component 1130 via a gap between the internal gasket element 171 and the air channel component 1170 or the like. In this second embodiment, the air channel component 1170 exemplarily extends along the whole length of the liquid reservoir 1100 of the syringe refiller 200. However, also other syringe refillers 1200 can be realized with other air channelling systems 1169, especially with air channelling systems 1169 that have other components and/or which do not transport air into the hollow body 1135 of a plunger component 1130. For example, such other air channelling systems 1169 can have own compartments or reservoirs within a syringe refiller for sucked air to be stored or contained in.

The air channel component 1170 comprises a first channel opening 1170-1 and a second channel opening 1170-2, the first channel opening 1170-1 being arranged within the hollow body 1135, wherein the second channel opening 1170-2 is connected to a second syringe needle element 180, adapted for the suction of air. An advantage of that may be that the flow of air from the liquid reservoir of an electronic smoking device to the hollow body 1135 of the syringe refiller 1200 is ameliorated since the second channel opening 1170-2 can directly be connected to an air outlet of the liquid reservoir of the electronic smoking device via the second syringe needle element 180 which in this second embodiment is identical to the first syringe needle element 1160. Also, in this second embodiment, the first syringe needle element 1160 and the second syringe needle element 180 are exemplarily realized as hollow needles that extend along a line that is parallel to a center line CL of the liquid reservoir 1100 of the syringe refiller 200 respectively. However, in this second embodiment, the first and the second syringe needle elements 1160, 180 are shorter than the first syringe needle element 160 as realized in the first embodiment of the syringe refiller 1200 respectively. Even though the plunger 1140 is configured slidable along the air channel component 1170, the length of the air channel component 1170 is chosen such that the first channel opening 1170-1 is arranged within the hollow body 1135, notwithstanding the position of the plunger 1140 relative to the air channel component 1170. Therefore, even when the plunger component 1130 is entirely pulled to the back end of the syringe refiller 1200, so when the syringe filler 1200 is in a first state in which the plunger 1140 is closest to the first end 1101 of the liquid reservoir 1100, the first channel opening 1170-1 is arranged within the hollow body 1135 of the syringe refiller 1200.

In this second embodiment, the connection between the second channel opening 1170-2 of the air channel component 1170 and the second syringe needle element 180 is realized within a stopper element 190 that is arranged at the second end 1102 of the liquid reservoir 1100. In more detail, the syringe refiller 1200 further comprises a stopper element 190 arranged at the second end 1102 with the second opening 1120 arranged within the stopper element 190 and an inlay collar 195 arranged at the first end 1101 of the liquid reservoir 1100, wherein the first opening 1110 is arranged within the inlay collar 195. An advantage of that may be that the manufacturing of the syringe refiller 1200 is eased and more cost-efficient since substantial components of the same can simply be attached to the liquid reservoir 1100 via a push-fit or via a press-fit connection. Furthermore, the assembling and disassembling of the syringe refiller 1200 is eased since the stopper element 190 and the inlay collar 195 can easily be plugged into the corresponding ends 1101, 1102 of the liquid reservoir 1100 and de-plugged of the same.

In this second embodiment, the inlay collar 195 has the shape of a plug that is plugged into the first end 1101 of the tube-shaped liquid reservoir 1100. In more detail, the inlay collar 195 comprises two substantially cylindrical portions 195-1, 195-2 that have a different diameter respectively and together integrally form the inlay collar 195. The first cylindrical portion 195-1 has a smaller diameter than the second cylindrical portion 195-2 and fits directly into the tube of the liquid reservoir 1100, wherein the peripheral walls of the first cylindrical portion 195-1 are in direct contact with the inner walls 1105 of the tube-shaped liquid reservoir 1100. The second cylindrical portion 195-2 of the inlay collar 195 rests on the first end 1101, that is on the outer walls of the liquid reservoir at the first end 1101 of the same, when the first cylindrical portion 195-1 is fully inserted into the tube-shaped liquid reservoir 1100. In this second embodiment, the inlay collar 195 comprises the first opening 1101 that in this second embodiment exemplarily has a diameter that varies along the width of the inlay collar 195. At a first end of the inlay collar 195 which faces the rear side of the plunger 1140, the diameter of the first opening 1101 is larger than the diameter of a central part of the first opening 1101. Thus, the inlay collar 195 can receive a corresponding counter part which is arranged at the rear side of the plunger 1140 in the first state of the syringe refiller 1200, in which the plunger 1140 is closest to the first end 1101 of the liquid reservoir 1100. Furthermore, the inlay collar 195 comprises an inlay gasket 1132 that is arranged close to a central portion of the inlay collar 195 in an area of the inlay collar 195 that has a diameter which is as large as the diameter at the first end of the inlay collar 195. The inlay gasket 1132 is adjusted to the diameter of the tube or rod shaped hollow body 1135 of the plunger component 1130, so that the inlay gasket 1132 seals the liquid reservoir 1100 on the one hand while on the other hand the plunger component 1130 is still moveable longitudinally within the liquid reservoir 1100. However, also other embodiments of syringe refillers 1200 with other or without inlay collars can be realized, especially with other inlay collars that comprise other openings or tunnels as the one shown in Figure 3 and described hereinbefore.

In this second embodiment, the stopper element 190 arranged at the second end 1102 comprises three substantially cylindrical portions 190-1, 190-2 , 190-3 that have a different diameter respectively and together integrally form the stopper element 190. The first cylindrical portion 190-1 has a smaller diameter than the second cylindrical portion 190-2 and fits directly into the tube of the liquid reservoir 1100, wherein the peripheral walls of the first cylindrical portion 190-1 are in direct contact with the inner walls 1105 of the tube-shaped liquid reservoir 1100. The second cylindrical portion 190-2 rests on the second end 1102, so on the outer walls of the liquid reservoir 1100 at the second end 1102 of the same, when the first cylindrical portion 190-1 is fully inserted into the tube-shaped liquid reservoir 1100. The third cylindrical portion 190-3 protrudes from the second cylindrical portion 190-2 towards a direction pointing away from the liquid reservoir 1100. It has the smallest diameter of the three substantially cylindrical portions 190-1, 190-2, 190-3 but also the greatest thickness or width.

In this second embodiment, the stopper element 190 comprises the second opening 1102 that in this second embodiment exemplarily has a diameter that varies along the width of the stopper element 190. The second opening 1120 has a narrowing cross-section with the largest diameter on the side of the stopper element 190 that is facing the liquid reservoir 1100 with the plunger 1140 therein. Thus, in this second embodiment, a first fraction of the second opening 1120 has a cross-section that has the shape of a nozzle or of a funnel allowing for a larger amount of liquid within the first chamber 1100a of the liquid reservoir 1100 to enter the second opening 1120. Expressed in other words, the shape of the cross section of the first fraction of the second opening 1120 resembles the shape of a screw with the screw head pointing towards the plunger 1140. Therefore, a first part of the first fraction of the second opening 1120 has the shape of a funnel. A second, following part of the first fraction of the second opening 1120 has the shape of a prismatic cylinder, wherein a third part of the first fraction of the second opening 1120 following this prismatic cylinder has a paraboloid shape with a hole at the tip of the paraboloid. From this part of the first fraction of the second opening 1120 on, a second fraction of the second opening 1120 extends within the stopper element 190. This second fraction has a cross-section that has a constant diameter, the second fraction being adapted to receive the first syringe needle element 1160. In this second embodiment, the second opening 1120 is exemplarily not arranged in a center portion of the stopper element 190 but is arranged displaced with regard to the center line CL of the liquid reservoir 1100 within the stopper element 190. While the second fraction and the third part of the first fraction of the second opening 1120 are realized within the third cylindrical portion 190-3 of the stopper element 190, the first and second part of the first fraction of the second opening 1120 are realized within the first and second cylindrical portions 190-1, 190-2 of the stopper element 190.

In this second embodiment, the first and the second syringe needle elements 1160, 180 are arranged within the stopper element 190 and protrude from the stopper element 190 along a direction that is pointing away from the liquid reservoir 1100 respectively. An advantage of that may be that the first and the second syringe needle elements 1160, 180 can simultaneously be connected or attached to corresponding openings of an electronic smoking device or of a liquid reservoir of an electronic smoking device that needs to be refilled. Therefore, in such an embodiment, the syringe refiller 1200 - when connected to an electronic smoking device/liquid reservoir - provides for a closed refill system. Furthermore, the liquid reservoir of an electronic smoking device can easily and quickly be connected to the syringe refiller 1200 which allows for a refill of the liquid reservoir, enabling a pressure equalization and assuring that a leakage or spilling of liquid is prevented.

In this second embodiment, the liquid reservoir 1100 of the syringe refiller 1200 comprises a third opening 121 which is also arranged within the second end 1102 of the liquid reservoir 1100. In more detail, in this second embodiment, also the third opening 121 is arranged within the stopper element 190, wherein the third opening 190 extends along the centre line CL of the liquid reservoir 1100. The third opening 121 is substantially identical to the second opening 1120 and also comprises a nozzle or funnel shaped first fraction and a following second fraction that has a cross-section with a constant diameter. Also the first fraction of the third opening 121 comprises a first part that has the shape of a funnel. A second, following part of the first fraction of the third opening 121 has the shape of a prismatic cylinder, wherein a third part of the first fraction of the third opening 121 following this prismatic cylinder has a paraboloid shape with a hole at the tip of the paraboloid. The first and the second syringe needle elements 1160, 180 are arranged within the second fractions of the second and third opening 1120, 121 arranged within the second end 1102 respectively. In this second embodiment, the stopper element 190 further comprises a reception cavity 191 that receives the fraction 172 of the air channel component 1170 that comprises the second channel opening 1170-2. An advantage of that may be that the air channelling system 1169 and especially the air channel component 1170 are fixed within the syringe refiller 1200 which provides the syringe refiller 1200 with a strong construction. Furthermore, the stopper element 190 is well-suited to provide for an airtight connection between the air channel component 1170 or the second channel opening 1170-2 and the second syringe needle element 1160, 180. Expressed in other words, the air channel component 1170 of the air channelling system 1169 protrudes into the first and second part of the first fraction of the third opening 121 within the stopper element 190. Thus, in this second embodiment, the first and second part of the first fraction of the third opening 121 exemplarily form/represent the reception cavity 191 within the stopper element 190. Therefore, the second channel opening 1170-2 of the air channel component 1170 is arranged within the stopper element 190 in front of the paraboloid-shaped third part of the first fraction of the third opening 1120.

Via the paraboloid-shaped third part of the first fraction of the third opening 1120, the second channel opening 1170-2 is connected to the second syringe needle element 180 which is adapted for the suction of air. Therefore, the second channel opening 1170-2 is not physically connected to the second syringe needle element 180 but the connection is provided via an airtight gap between the second channel opening 1170-2 and the second syringe needle element 180. Air that is exiting the second syringe needle element 180 and transported into the gap is pushed directly into the second channel opening 1170-2 of the air channel component 1170. Since the air channel component 1170 is airtight and strongly affixed or fitted to the stopper element 191, the air exiting the second syringe needle element 180 cannot escape the stopper element 191 without entering the second channel opening 1170-2. However, also other embodiments of syringe refillers 1200 can be carried, comprising air channel components 1170 with second channel openings 1170-2 that are physically connected to the second syringe needle element 180 respectively.

In this second embodiment, the syringe refiller 1200 further comprises a child-safety cap 182 arranged at the second end 1102 of the liquid reservoir 1100, being adapted to fully enclose the first syringe needle element 1160 in a locked state of the syringe refiller 1200 and to release the first syringe needle element 1160 in an unlocked state of the syringe refiller 1200. An advantage of that may be that such a child-safety cap 182 prevents children from getting in contact with the first syringe needle element 1160, avoiding injuries or a misuse of the syringe refiller 1200. Furthermore, the child-safety cap 182 also allows for an improved transportation of the syringe refiller 1200, for example within a users pocket or the like.

Moreover, the stopper element 190 comprises a spring element 192, wherein the child-safety cap 182 is configured slidable along the stopper element 190 and wherein the child-safety cap 182 is interacting with the spring element 192 when slid along the stopper element 190. An advantage of that may be that such a slidable child-safety cap 182 can easily be realized and easily but efficiently allows for the provision of a child-safe locked state and an unlocked state in which the syringe refiller 1200 is usable for a refill process. In this second embodiment, the child-safety cap 182 has the outer shape/outline of the mouthpiece of a flute or of a pipe. In more detail, the child-safety cap 182 has a cylindrical base part 183 with grooves and protrusions, so with groove-like depressions 183-1 on two opposing sides of the cylindrical base part 183 thereon that allow for a better grip of the child-safety cap 182 and which allow for the child-safety cap 182 to be easily pushed and pulled along the stopper element 190. A nozzle part 184 extends from the cylindrical base part 183 in a direction pointing away from the liquid reservoir 1100 of the syringe refiller 1200. The nozzle part 184 of the child-safety cap 182 comprises a circular nozzle cavity 184-1 at a tip of the nozzle part 184 of the child-safety cap 182.

The child-safety cap 182 further comprises a first hole 182-1 arranged such that the first syringe needle element 1160 pierces through the child-safety cap 182 when the child-safety cap 182 is pulled back in the unlocked state of the syringe refiller 200. An advantage of that may be that the first syringe needle element 160 can easily be covered by the child-safety cap 182 in a locked state and easily be released in an unlocked state, simply by sliding the child-safety cap 182 along the stopper element 190. In this second embodiment of the syringe refiller 1200, the child-safety cap 182 is further adapted to fully enclose the second syringe needle element 180 in a locked state and to release the second syringe needle element 180 in an unlocked state. An advantage of that may be that both the first syringe needle element 1160 and the second syringe needle element 180 can be covered in a child-safe locked state of the syringe refiller 1200. This ameliorates the usability and the transportability of the syringe refiller 1200 and makes it safer for children that get in contact with the syringe refiller 1200. In order to allow for the child-safety cap 182 of the syringe refiller 1200 to enclose and release the (first and) second syringe needle element 180, the child-safety cap 182 further comprises a second hole 182-2 arranged such that the second syringe needle element 180 pierces through the child-safety cap 182 when the syringe refiller 200 is in the unlocked state. An advantage of that may be that both syringe needle elements 1160, 180 can be enclosed and released via the child-safety cap 182 simultaneously. In this second embodiment, the first and the second hole 182-1, 182-2 are arranged within the nozzle cavity 184-1 at the tip of the nozzle part 184. The first hole 182-1 is aligned with the first syringe needle element 1160 and the second hole 182-2 is aligned with the second syringe needle element 180.

In this second embodiment, the spring element 192 is realized as a spring that comprises a plurality of windings of which a first winding is arranged within a corresponding ring-shaped circular cavity that is arranged within the third cylindrical portion 190-3 of the stopper element 190. The corresponding circular cavity is concentrically arranged around the second fraction of the third opening 121 so that the windings of the spring element 192 that is arranged within the ring-shaped circular cavity coils around a fraction of the second syringe needle element 180. The child-safety cap 182 further comprises an internal fit that corresponds to the outlines and the shape of the third cylindrical portion 190-3 of the stopper element 190. Therefore, the fit can be slid along the third cylindrical portion 190-3 of the stopper element 190 in order transfer the syringe refiller 1200 from a locked state into an unlocked state, exposing the first and the second syringe needle elements 1160, 180. The stopper element 190 further comprises a cylindrical cavity arranged within the second cylindrical portion 190-2 and extending from the second cylindrical portion 190-2 into a fraction of the first cylindrical portion 190-1. This cylindrical cavity enlarges the surface of the third cylindrical portion 190-3 and thereby the path along that the internal fit of the child-safety cap 182 can be slid. Expressed in other words, the cylindrical cavity allows for the child-safety cap 182 with its internal fit to be fully slid onto the third cylindrical portion 190-3 of the stopper element 190 until the inner rim portions of the first and the second hole 182-1, 182-2 within the child-safety cap 182 are in direct contact with the outer surface of the third cylindrical portion 190-3 of the stopper element 190 and until the spring element 192 is fully compressed. A rear wall of the nozzle cavity comprises internal protrusions for the reception of the last winding of the spring element 192 in a compressed state of the same.

When the child-safety cap 182 is slid along the stopper element 190 and moved towards the liquid reservoir 1100 in order to transfer the syringe refiller 1200 from a locked state into an unlocked state, the movement is not hindered as long as the child-safety cap 182 is not in contact with the spring element 192. As soon as the rear wall of the circular nozzle cavity 184-1 at the tip of the nozzle part 184 of the child-safety cap 182 contacts the spring element 192, the spring element 192 will exert a force on the child-safety cap 182, pushing the child-safety cap 182 into the opposite direction. For a transfer of the syringe refiller 1200 from a locked state into an unlocked state, the force exerted by the spring element 192 needs to be surmounted by a user and the child-safety cap 182 needs to be pushed further until it contacts the front side of third cylindrical portion 190-3 of the stopper element 190.

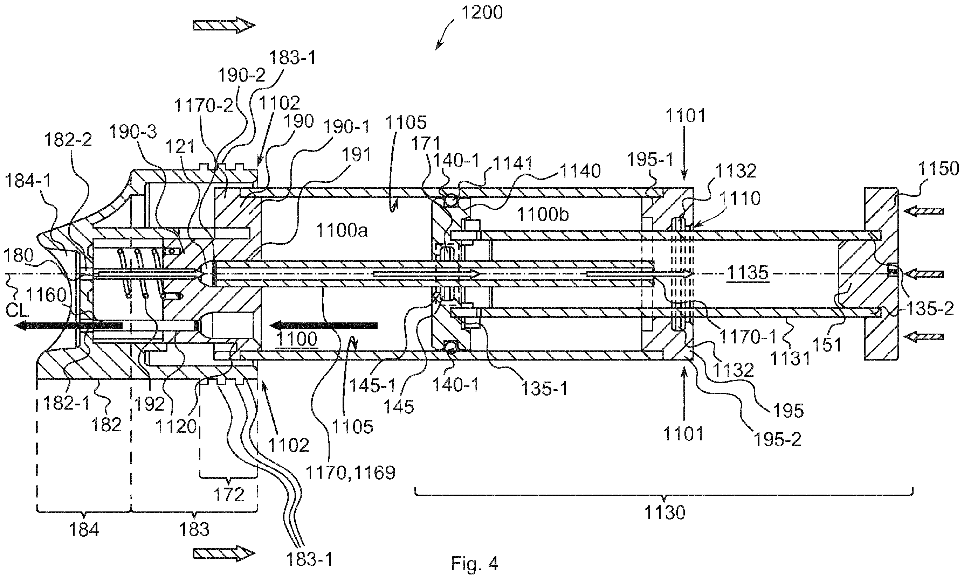

In Figure 4, a schematic vertical cross-sectional illustration of the second embodiment of a syringe refiller 1200 is shown in use. Expressed in other words, Figure 4 shows the second embodiment of a syringe refiller 1200 as shown in Figure 3 and as described hereinbefore, in use. In more detail, Figure 4 shows the syringe refiller 1200 in a locked state. In this locked state, the child-safety cap 182 encloses the first and the second syringe needle elements 1160, 180 so that the first and the second syringe needle elements 1160, 180 are fully arranged within the child-safety cap 182. For a usage of the syringe refiller 1200 in order to refill the liquid reservoir of an electronic smoking device, the syringe refiller 1200 is transferred from the locked state into an unlocked state by pulling or pushing back the child-safety cap 182. The force that is exerted to the child-safety cap 182 in Figure 4 is indicated by the hatched arrows that are shown close to the circular grooves and protrusions, so to the groove-like depressions 183-1 of the child-safety cap 182. Expressed in other words, a force is applied to the child-safety cap 182 by pushing or pulling the child-safety cap 182 into a direction that is pointing from the second end 1102 to the first end 1101 of the liquid reservoir 1100.

When the child-safety cap 182 is fully pulled or pushed towards the second end 1102 so that the syringe refiller 1200 is transferred from a locked state into an unlocked state, the first and the second syringe needle elements 1160, 180 are entirely exposed and the syringe refiller 1200 can be used to refill the liquid reservoir of an electronic smoking device, for example the liquid reservoir 34 of the electronic smoking device 10 shown in Figure 1. For a refilling of this liquid reservoir 34, the first and the second syringe needle elements 1160, 180 are inserted into the separate openings 33-1, 33-2 of the outer refill interface 33 of the electronic smoking device 10.

However, other electronic smoking devices which are refillable via the syringe refiller 1200 can also comprise other refill openings. For example, in some other embodiments of electronic smoking devices, the first and the second syringe needle elements 1160, 180 can also be inserted into refill openings of an electronic smoking device which are arranged within an inner component of the electronic smoking device or of a liquid reservoir of an electronic smoking device.

When the syringe needle elements 1160, 180 are inserted into the corresponding openings, the handle 1150 of the syringe refiller 1200 is pushed into a direction pointing from the handle 1150 to the second end 1102 of the syringe refiller 1200. In Figure 4, this is indicated by three hatched arrows that point onto the handle 1150. The pushing action will move the plunger 1140 towards the second end 1102 of the syringe refiller 1200, reducing the volume of the first chamber 1100a of the liquid reservoir 1100 and pushing out the liquid contained within the first chamber 1100a via the second opening 1120 and the first syringe needle element 1160 which protrudes through the first hole 182-1 in the child-safety cap 182. The flow of liquid from the first chamber 1100a into the second opening 1120 and out of the syringe refiller 1200 in Figure 4 is indicated by dark/black arrows.

When the liquid is pushed out of the first chamber 1100a and into the liquid reservoir 34 of the electronic smoking device 10, air is pushed out of the liquid reservoir 34 and into the second syringe needle element 180. Via the second syringe needle element 180, the air passes the third opening 121 within the stopper element 190 and enters the air channel component 170 via the second channel opening 1170-2. Via the air channel component 1170, the air flows into the hollow body 1135 of the plunger component 1130, exiting the air channel component 1170 via the first channel opening 1170-1. In Figure 4, the flow of air is indicated by white arrows.

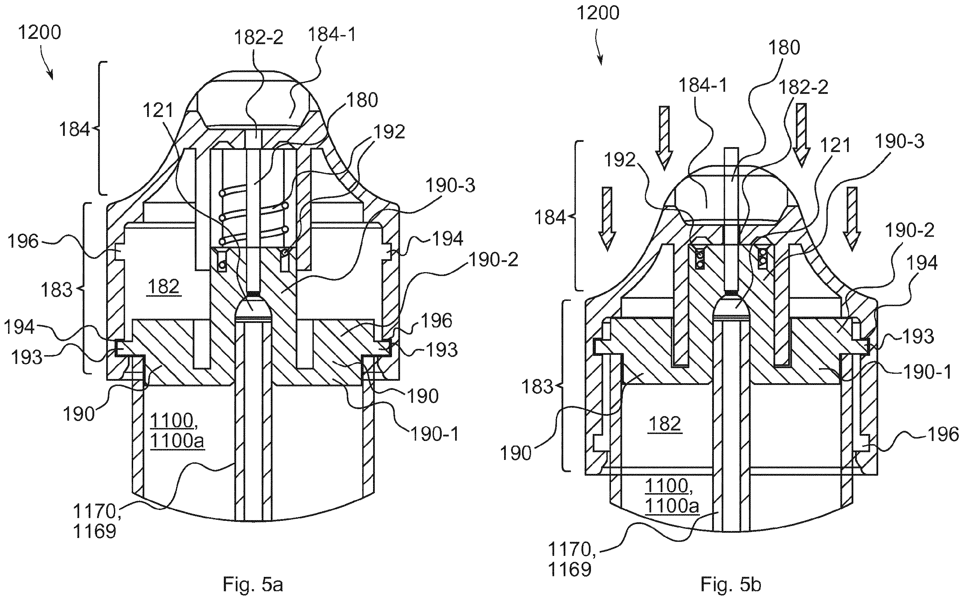

Figure 5a is a schematic horizontal cross-sectional illustration of the front part of the second embodiment of a syringe refiller 1200 in a locked state. Expressed in other words, Figure 5a illustrates a horizontal cross-section of a fraction of the second embodiment of a syringe refiller 1200, showing a cross-section through the child-safety cap 182, the stopper element 190 and through a fraction of the liquid reservoir 1100 with the air channel component 1170 arranged therein from above. Thus, the cross-section shown in Figure 5a shows a cut through the second syringe needle element 180, but not through the first syringe needle element 1160 as in the vertical cross-sections shown in Figure 3 and 4.

As can be seen in Figure 5a, the stopper element 190 comprises two releasable connection elements 193 on outer sides of the stopper element 190, the releasable connection elements 193 being adapted to engage with first corresponding engagement elements 194 arranged within the child-safety cap 182 in an unlocked state of the syringe refiller 1200. An advantage of that may be that the child-safety cap 182 can easily but nevertheless releasable be transferred from a locked state into an unlocked state, since the releasable connection elements 193 of the stopper element 190 will snap in and engage with the first corresponding engagement elements 194 when the child-safety cap 182 is pulled back towards the liquid reservoir 1100 until the releasable connection elements 193 of the stopper element 190 arrive at the first corresponding engagement elements 194 arranged within the child-safety cap 182. Such a state is shown in Figure 5b. In more detail, Figure 5b shows a schematic horizontal cross-sectional illustration of the front part of the second embodiment of a syringe refiller 1200 in an unlocked state, so with the second syringe needle element 180 protruding out of the second hole 182-2 of the child-safety cap 182. Expressed in other words, also Figure 5b illustrates a horizontal cross-section of a fraction of the second embodiment of a syringe refiller 1200, showing a cross-section through the child-safety cap 182, the stopper element 190 and a fraction of the liquid reservoir 1100 with the air channel component 1170 arranged therein from above. Thus, also the cross-section shown in Figure 5b shows a cut through the second syringe needle element 180, but not through the first syringe needle element 1160 as in the vertical cross-sections shown in Figure 3 and 4.

In this second embodiment, second corresponding engagement elements 196 are arranged within the child-safety cap 182, adapted to engage with the releasable connection elements 193 in a locked state of the syringe refiller 1200. An advantage of that may be that also in the locked state of the syringe refiller 1200, the child-safety cap 182 is fixed to the stopper element 190 and therefore immovably attached to the same. Such a state of the syringe refiller 1200 is shown in Figure 5a, where the second corresponding engagement elements 196 are in an engaged state with the releasable connection elements 193 on the outer sides of the stopper element 190. In this second embodiment of the syringe refiller 1200, the releasable connection elements 193 are realized as retaining lugs and the first corresponding engagement elements 194 are realized as fixation holes, the retaining lugs and the fixation holes together form snap-action connections respectively. An advantage of that may be that retaining lugs and corresponding fixation holes can easily be realized in a small dimension, are cost-efficient and provide for a strong connection between the child-safety cap 182 and the stopper element 190.

Moreover, also the second corresponding engagement elements 196 are exemplarily realized as fixation holes in this second embodiment of the syringe refiller 1200. In more detail, in this second embodiment, the first and second corresponding engagement elements 194, 196 are realized as fixation notches or edges which are arranged on an inner side of the child-safety cap 182 respectively.

In Figure 5a, the syringe refiller 1200 is shown in a locked state in that the first and second syringe needle elements 1160, 180 are enclosed by the child-safety cap 182. The spring element 192 is fully expanded and the releasable connection elements 193, so the retaining lugs are in an engaged state with the second corresponding engagement elements 196, so the corresponding fixation holes that are arranged on an inner side of the side walls of the child-safety cap 182. In this second embodiment, the child-safety cap 182 comprises a flexible and elastic material, in more detail a flexible and elastic plastic material. However, also other embodiments of syringe refillers 1200 with other child-safety caps 182 can be realized and come to use.

In order to release the releasable connection elements 193 from the second corresponding engagement elements 196 and to transfer the syringe refiller 1200 from the locked state to an unlocked state, the groove-like depressions 183-1 on the upper and the lower side of the child-safety caps 182 as shown in Figures 3 and 4 are pressed in a direction pointing towards one another respectively, for example with a pinch movement using two fingers of a hand, decreasing the distance between the portions of the child-safety caps 182 that comprise the groove-like depressions 183-1. This pinch movement or compression of the upper and lower sides of the child-safety caps 182 will bend the sidewalls of the child-safety cap 182 with the second corresponding engagement elements 196 - realized as the corresponding fixation holes - to an outward direction respectively. Expressed in other words, the pinch movement will increase the distance between the second corresponding engagement elements 196, releasing the retaining lugs of the child-safety cap 182 from the corresponding fixation holes of the stopper element 190. Simultaneously to the pinch movement or the pinch action, the child-safety cap 182 is pushed or pulled in a direction that is indicated by the hatched arrows shown in Figure 5b, so into a direction pointing towards the liquid reservoir 1100 of the syringe refiller 1200. The released child-safety cap 182 is slid along the stopper element 190, compressing the spring element 192, until the retaining lugs snap into the first corresponding engagement elements 194 of the child-safety cap 182. When this is achieved, the syringe refiller 1200 successfully has been transferred into the unlocked state. The syringe refiller 1200 is then ready to be used in order to refill the liquid reservoir of an electronic smoking device.

After use, the syringe refiller 1200 can be retransferred into the locked state by performing the aforementioned pinch movement or pinch action again, causing the retaining lugs of the stopper element 190 to be released from the first corresponding engagement elements 194 of the child-safety cap 182. When the retaining lugs are released from the first corresponding engagement elements 194, the spring will decompress and push or force the child-safety cap 182 along the sliding surface of the stopper element 190, causing the child-safety cap 182 to slide along the stopper element 190 until the retaining lugs engage with the second corresponding engagement elements 196 again as shown in Figure 5a.

In this second embodiment, the releasable connection elements 193 are realized as retaining lugs or protrusions which protrude from the stopper element 190. However, also other releasable connection elements 193 can be realized which can also be arranged in other positions, for example on an inner side of the child-safety cap 182, protruding inwardly from the inner sides of the child-safety cap 182. Furthermore, it is also possible to realize other embodiments of syringe refillers 1200 with only one or more than two releasable connection elements 193. Moreover, the first and second corresponding engagement elements 194, 196 are realized as fixation holes, fixation cavities or fixation notches which are arranged displaced from one another within an inner wall of the child-safety cap 182. However, also other first and second corresponding engagement elements 194, 196 can be realized which can also be arranged within other components of the syringe refiller 1200, for example within the stopper element 190. Furthermore, it is also possible to realize other embodiments of syringe refillers 1200 with only one or more than two first and second corresponding engagement elements 194, 196 respectively. Moreover, in this second embodiment, the first and second corresponding engagement elements 194, 196 are arranged within the inner sides of the sidewalls of the cylindrical base part 183 of the child-safety cap 182 respectively. Furthermore, along the length of the syringe refiller 1200, seen from the first end 1101 of the syringe refiller 1200 to the nozzle cavity 184-1 of the child-safety cap 182, the first and second corresponding engagement elements 196 are arranged having a distance between them, wherein the first corresponding engagement elements 194 are arranged behind the second corresponding engagement elements 196 along the aforementioned length.

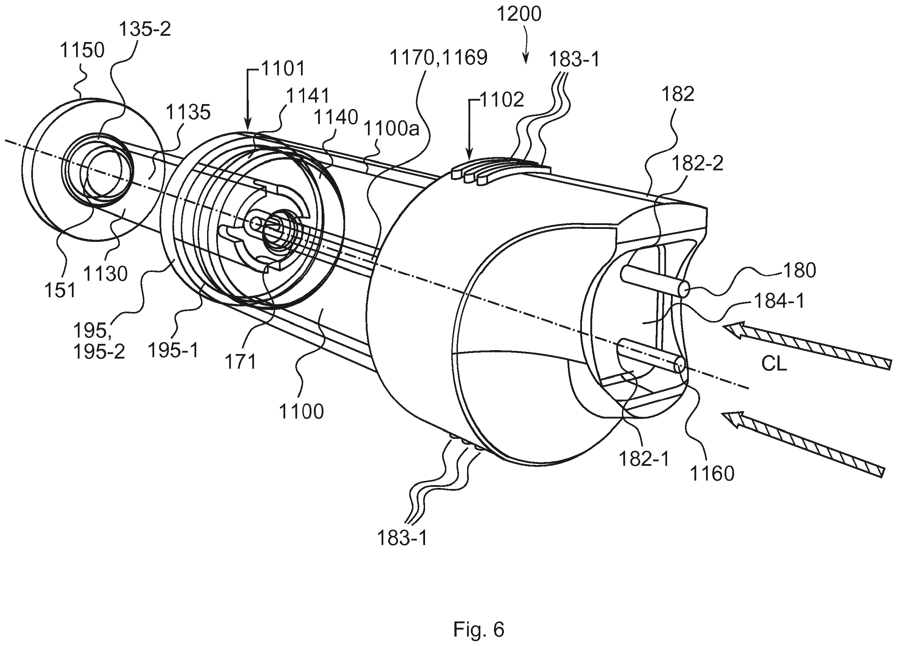

Figure 6 shows a schematic perspective view of the second embodiment of a syringe refiller 1200 in an unlocked state. In more detail, Figure 6 shows the second embodiment of a syringe refiller 1200 from a front perspective with the child-safety cap 182 that has been pushed towards a direction pointing from the second end 1102 of the syringe refiller 1200 to the first end 1101 of the syringe refiller 1200 so that the syringe refiller 1200 is in an unlocked state. The hatched arrows in Figure 6 indicate the direction the child-safety cap 182 has been pushed in so that the first and the second syringe needle elements 180, 1160 are visible to the viewer, protruding from the child-safety cap 182 along a direction that is parallel to the center line of the syringe refiller 1200.

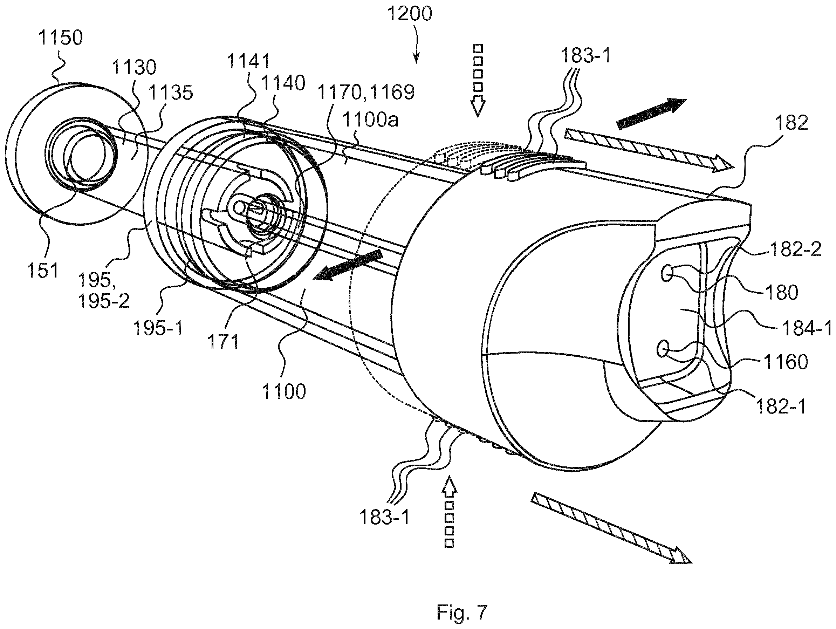

In Figure 7, a schematic perspective view of the second embodiment of a syringe refiller 1200 in a locked state is shown. In more detail, Figure 7 indicates the transfer of the syringe refiller 1200 from the unlocked state into the locked state. The dotted arrows in Figure 7 indicate the aforementioned pinch movement/action performed to the groove-like depressions 183-1 on the upper and lower side of the child-safety cap 182. This pinch movement/action will bend the side portions of the child-safety cap 182 in an outward direction, causing the child-safety cap 182 to be released from the stopper element (not visible in Figure 7). In Figure 7, this bending is indicated via black arrows. As in the unlocked state, the child-safety cap 182 is pre-loaded via the spring element (not shown in Figure 7), the release of the child-safety cap 182 also releases the spring element (not visible in Figure 7), causing the spring element to relax/decompress, pushing the child-safety cap 182 along the stopper element in a direction that in Figure 7 is indicated by hatched arrows until the syringe element 1200 is fully transferred into the locked state.

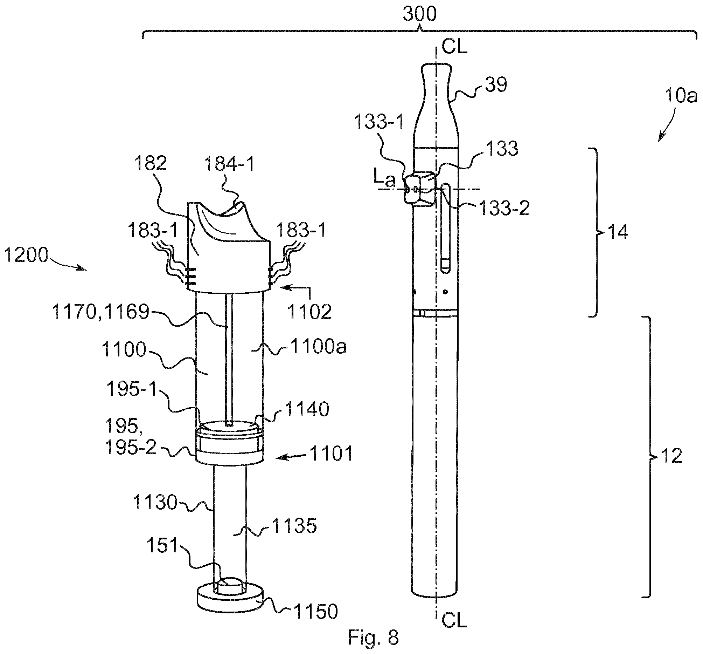

Figure 8 shows an embodiment of an electronic smoking device system 300 with an embodiment of an electronic smoking device 10a and the second embodiment of a syringe refiller 1200. In more detail, Figure 8 shows an embodiment of an electronic smoking device system 300 that comprises an electronic smoking device 10a with two refill openings 133-1, 133-2 and the second embodiment of a syringe refiller 1200 as described hereinbefore, adapted to refill the liquid reservoir of the electronic smoking device via the two refill openings 133-1, 133-2. An advantage of such a system 300 may be that the electronic smoking device can be refilled and therefore reused when its liquid reservoir is emptied. It does not need to be exchanged which is cost-efficient, ecologically beneficial and more pleasant to the consumer.

The embodiment of an electronic smoking device 10a shown in Figure 8 is substantially identical to the electronic smoking device 10 as shown in Figure 1. Thus, also in this embodiment, the electronic smoking device 10a exemplarily comprises the same atomizer/liquid reservoir portion 14 and the same power supply portion 12 attached thereto. However, the electronic smoking device 10a shown in Figure 8 exemplarily further comprises a nozzle shaped mouthpiece 39 that is attached onto the liquid reservoir 34, comprising an opening that is aligned to the air inhalation port 36 arranged within the liquid reservoir 34.

In this embodiment of the electronic smoking device 10a, the two refill openings 133-1, 133-2 are arranged within an outer refill interface 133 which protrudes from the liquid reservoir 34 and in this embodiment exemplarily has a trapezoid shape. The refill openings 133-1, 133-2 are arranged within a line La that is perpendicular to the longitudinal center line CL of the electronic smoking device 10a. Expressed in other words, the line La, the refill openings 133-1, 133-2 of the outer refill interface 133 are arranged in, is perpendicular to the length of the electronic smoking device 10a. However, also other electronic smoking devices with other liquid reservoirs, with other outer refill interfaces 133 and with other refill openings 133-1, 133-2 within such other outer refill interfaces 133 can be realized.

When the liquid reservoir 34 of the electronic smoking device 10a of the electronic smoking device system 300 is emptied, the syringe refiller 1200 of the electronic smoking device system 300 can be used to refill the liquid reservoir of the electronic smoking device 10a. In order to perform such a refill, the first and second syringe needle elements (not shown in Figure 8) are inserted in to the refill openings 133-1, 133-2 of the outer refill interface 133 of the electronic smoking device 10a and the syringe refiller 1200 is actuated.

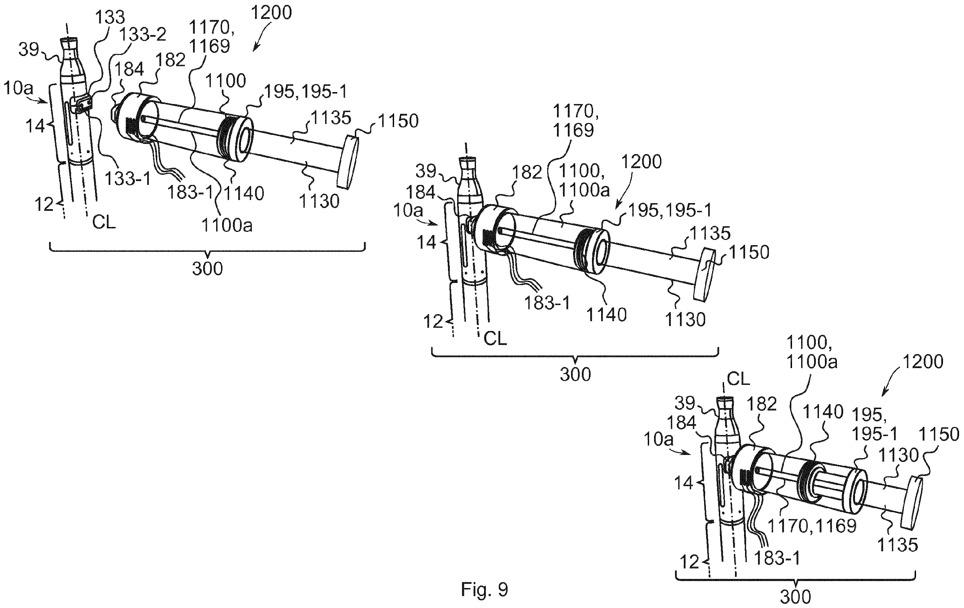

In Figure 9, a refilling of the embodiment of the electronic smoking device 10a of the electronic smoking device system 300 is shown in three images, using the second embodiment of a syringe refiller 1200. On the left in Figure 9, the nozzle part 184 of the child-safety cap 182 of the syringe refiller 1200 is approached to the outer refill interface 133 of the electronic smoking device 10a. The nozzle part 184 and the nozzle cavity 184-1 therein correspond to the outer refill interface 133 of the electronic smoking device 10a. Therefore, in this embodiment, the trapezoidal outer refill interface 133 fits into the nozzle cavity 184-1 of the nozzle part 184. However, in other embodiments, other fits or connections between the outer refill interface 133 of an electronic smoking device and a nozzle cavity or the syringe needle elements of a syringe refiller can be realized.

In the middle of Figure 9, the trapezoidal outer refill interface 133 is inserted into the nozzle cavity 184-1 of the nozzle part 184 and the syringe refiller 1200 is pushed against the electronic smoking device 10a, so that the child-safety cap 182 is pushed back along the stopper element (not visible in Figure 9) until the syringe refiller 1200 is transferred from the locked state into the unlocked state. In that unlocked state, the first and second syringe needle elements 1160, 180 are exposed and - due to the fit between the nozzle cavity 184-1 and the outer refill interface 133 - directly inserted into the refill openings 133-1, 133-2 of the outer refill interface 133 of the electronic smoking device 10a.

On the right in Figure 9, it is shown how a force is applied to the handle 1150, pushing the plunger component 1130 into the liquid reservoir 1100 of the syringe refiller 1200 in order to force liquid contained within the first chamber 1100a of the liquid reservoir 1200 out of the first chamber 1100a and into the liquid reservoir (not shown) of the electronic smoking device 10a via the first syringe needle element 1160. Air ousted out of the liquid reservoir of the electronic smoking device 10a enters the syringe refiller 1200 via the second syringe needle element 180 and will be transported to the hollow body 1135 of the plunger component 1130. The hollow body 1135 is not fully sealed so that in this second embodiment of the syringe refiller 1200, air within the hollow body 1135 can escape the same. When the liquid reservoir of the electronic smoking device 10a has been refilled via the syringe refiller 1200, the electronic smoking device 10a can be reused. However, in other embodiments of syringe refillers, the hollow body of the plunger component can also be fully sealed so that air within the hollow body 1135 cannot escape the same.

In the aforementioned embodiments, the plunger 140, 1140 of the syringe refiller 200, 1200 exemplarily comprises a stable rubber material. However, other plungers of other embodiments of syringe refillers can comprise other materials, for example a plastic material or an even harder material, as for example a wooden or a metallic material.

Furthermore, the liquid reservoir 100, 1100, the rod shaped shaft 131, 1131 of the plunger component 130, 1130 and the child-safety cap 182 can also comprise a plastic material, especially a thermoplastic material. However, the aforementioned components can also comprise other materials or be made of such other materials.

Moreover, in the aforementioned embodiments, the stopper element 190, the handle 150, 1150 and the inlay collar 195 are exemplarily realized as hard plastic components, comprising portions that have rubber in them. However, these components can also comprise or be made of other materials than the aforementioned.

It is provided a syringe refiller for an electronic smoking device, the syringe refiller comprising a liquid reservoir comprising a first end with a first opening for the reception of a plunger component and a second end with a second opening for a liquid to be pushed out of the liquid reservoir. Moreover, the syringe refiller comprises a plunger component comprising a plunger attached to a handle, the plunger being configured slidable along an inner wall of the liquid reservoir, wherein the plunger seals the liquid reservoir towards the first opening. The plunger component comprises a hollow body with a first end attached to the plunger and a second end attached to the handle. Further, the plunger comprises a hole for the insertion of an air channel component of an air channelling system. Furthermore, the syringe refiller comprises a first syringe needle element that is in connection with the second opening, so that liquid which is pushed out of the second opening via the plunger component flows through the syringe needle element. Moreover, the syringe needle element comprises an air channelling system which comprises an air channel component inserted into the hole of the plunger, adapted to transport air from outside of the syringe refiller into the hollow body, allowing for air to be sucked into the syringe refiller without interfering with liquid contained therein. Said air channel component comprises a first channel opening and at least one second channel opening, the first channel opening being arranged within the hollow body and the at least one second channel opening being arranged adjacent to the second opening within a sidewall at the second end of the liquid reservoir.

An advantage of that may be that an emptied liquid reservoir of an electronic smoking device can easily and quickly be refilled. Furthermore, the syringe needle element allows for a precise refilling of the liquid reservoir of an electronic smoking device without that liquid is dripped into the air tube via the fist syringe needle element. During the refill procedure, the pressure within the liquid reservoir of the electronic smoking device is equalized. Moreover, when connected to an electronic smoking device, the syringe refiller represents a closed refill system which prevents leakage or spilling of liquid since also the air or gas - which can contain liquid drops - that exits the liquid reservoir of an electronic smoking device during a refill process is recaptured by the air channelling system of the syringe refiller.

Preferably, the plunger component further comprises a hollow body with a first end attached to the plunger and a second end attached to the handle, the plunger comprising a hole for the insertion of an air channel component of the air channelling system adapted to transport air from outside of the syringe refiller into the hollow body. An advantage of that may be that air which is pushed out of the liquid reservoir of an electronic smoking device that is refilled can be released into the hollow body of the syringe refiller, which prevents liquid from being spilled together with air exiting the liquid reservoir of the electronic smoking device during a pressure equalization of the same.

In a preferred embodiment, the air channelling system comprises an air channel component and an internal gasket element, the internal gasket element being arranged within the hole of the plunger, providing for an airtight contact between the plunger and the air channel component, the plunger also being configured slidable along the air channel component. An advantage of that may be that liquid contained within the liquid reservoir is not mixed but strictly separated from air that is released into the hollow body during a pressure equalization within the liquid reservoir of an electronic smoking device that is refilled. Thus, the syringe refiller and especially the plunger component can be actuated without that the air channelling system of the syringe refiller is affected and without that air entering the syringe refiller and liquid exiting the syringe refiller is mixed. Furthermore, such a separation is necessary for the proper functioning of the syringe refiller, since an open connection between the liquid reservoir and the hollow body would cause liquid to be expelled via the first and the second syringe needle element during an actuation of the syringe refiller.

Preferably, the second channel opening is connected to a second syringe needle element, adapted for the suction of air. An advantage of that may be that the flow of air from the liquid reservoir of the electronic smoking device to the hollow body of the syringe refiller is ameliorated since the second channel opening can directly be connected to an air outlet of the liquid reservoir of the electronic smoking device via the second syringe needle element. This allows for the provision of a closed refill system for an electronic smoking device.

In a preferred embodiment, the syringe refiller further comprises a stopper element arranged at the second end with the second opening arranged within the stopper element and an inlay collar arranged at the first end of the liquid reservoir, wherein the first opening is arranged within the inlay collar. An advantage of that may be that the manufacturing of the syringe refiller is eased and more cost-efficient since substantial components of the same simply can be attached to the liquid reservoir via for example a push-fit connection. Furthermore, the assembling and disassembling of the syringe refiller is eased since the stopper element and the inlay collar can easily be plugged to the corresponding ends of the liquid reservoir and easily be de-plugged of the same.

In a furthermore preferred embodiment, the first and the second syringe needle elements are arranged within the stopper element and protrude from the stopper element along a direction that is pointing away from the liquid reservoir respectively. An advantage of that may be that the first and the second syringe needle elements can simultaneously be connected or attached to corresponding openings of an electronic smoking device or of a liquid reservoir of an electronic smoking device that needs to be refilled. Especially in such an embodiment, the syringe refiller connected to the electronic smoking device represents a closed refill system for the liquid reservoir of an electronic smoking device that can easily be connected to the same and that allows for a refill of the liquid reservoir, providing for a pressure equalization and assuring that a leakage or spilling of liquid is prevented.

Preferably, the stopper element further comprises a reception cavity that receives the fraction of the air channel component that comprises the second channel opening. An advantage of that may be that the air channelling system and especially the air channel component is fixed within the syringe refiller, which provides the syringe refiller with a strong construction. Furthermore, in such an embodiment, the stopper element is well-suited to provide for an airtight connection between the air channel component or the second channel opening and the second syringe needle element.

In a preferred embodiment, the syringe refiller further comprises a child-safety cap arranged at the second end of the liquid reservoir, being adapted to fully enclose the first syringe needle element in a locked state of the syringe refiller and to release the first syringe needle element in an unlocked state of the syringe refiller. An advantage of that may be that such a child-safety cap prevents children from getting in contact with the first syringe needle element avoiding injuries or a misuse of the syringe refiller. Furthermore, the child-safety cap also allows for an improved transportation of the syringe refiller, for example within a pocket or the like, preventing the needle from causing damage to human beings or objects.

Preferably, the stopper element comprises a spring element, wherein the child-safety cap is configured slidable along the stopper element and wherein the child-safety cap is interacting with the spring element when slid along the stopper element. An advantage of that may be that such a slidable child-safety cap can easily be realized and easily but efficiently allows for the provision of a child-safe locked state and an unlocked state in which the syringe refiller is usable for a refill process. Preferably, the child-safety cap is adapted to interact with the spring element when the child-safety cap is slid along the stopper element.

In a preferred embodiment, the child-safety cap further comprises a first hole arranged such that the first syringe needle element pierces through the child-safety cap when the child-safety cap is in the unlocked state of the syringe refiller. An advantage of that may be that the first syringe needle element can easily be covered by the child-safety cap in a locked state and easily be released in an unlocked state, simply by sliding the child-safety cap along the stopper element. Preferably, the stopper element comprises at least one releasable connection element on at least one outer side of the stopper element, the releasable connection element being adapted to engage with a first corresponding engagement element arranged within the child-safety cap in an unlocked state of the syringe refiller. An advantage of that may be that the child-safety cap can easily but nevertheless releasable be transferred from a locked state into an unlocked state, since the at least one releasable connection element of the stopper element will engage with the first corresponding engagement element when the child-safety cap is pulled back towards the liquid reservoir until the at least one releasable connection element of the stopper element arrives at the first corresponding engagement element arranged within the child-safety cap. Preferably, the releasable connection element is a retaining lug and the first corresponding engagement element is a fixation hole, the retaining lug and the fixation hole together form a snap-action connection. An advantage of that may be that retaining lugs and corresponding fixation holes can easily be realized in a small dimension, are cost-efficient and provide for a strong connection between the child-safety cap and the stopper element. Furthermore preferred, the first corresponding engagement element is realized as a fixation cavity or as a fixation hook.

In a preferred embodiment, a second corresponding engagement element is arranged within the child-safety cap, adapted to engage with the at least one releasable connection element in a locked state of the syringe refiller. An advantage of that may be that also in the locked state of the syringe refiller, the child-safety cap is affixed to the stopper element and therefore is immovably attached to the same. Preferably, when seen along the length of the child-safety cap, from the second end of the liquid reservoir on to the nozzle cavity, the first corresponding engagement element is arranged behind the second corresponding engagement element within an inner side of a sidewall of the child-safety cap.

Preferably, also the second corresponding engagement element is realized as a fixation hole, as a fixation cavity or as a fixation hook. In such an embodiment, a single retaining lug can come to use, adapted to engage with the first and the second fixation hole in order to define the locked state and the unlocked state of the syringe refiller.

In a furthermore preferred embodiment, the child-safety cap is further adapted to fully enclose the second syringe needle element in a locked state and to release the second syringe needle element in an unlocked state. An advantage of that may be that also the second syringe needle element can be covered in a child-safe locked state of the syringe refiller. This ameliorates the usability and the transportability of the syringe refiller and makes it safer for children that get in contact with the syringe refiller.

Preferably, the child-safety cap further comprises a second hole arranged such that the second syringe needle element pierces through the child-safety cap when the syringe refiller is in the unlocked state. An advantage of that may be that both syringe needle elements can be enclosed and released via the child-safety cap simultaneously.

Furthermore, an electronic smoking device system is provided, the electronic smoking device system comprising an electronic smoking device with at least one refill opening and a syringe refiller. An advantage of such a system may be that the electronic smoking device can be refilled and therefore reused when its liquid reservoir is emptied. It does not need to be exchanged which is cost-efficient, ecologically beneficial and more pleasant to the consumer. Moreover, the advantages that have been mentioned in relation to the features described above also apply for the syringe refiller that comes to use in the electronic smoking device system. Preferably, the at least one refill opening of the electronic smoking device is arranged within an outer refill interface that protrudes from the liquid reservoir of the electronic smoking device. Furthermore preferred, the outer refill interface comprises a body portion that corresponds to a nozzle cavity of the child-safety cap of the syringe refiller.

Preferably, the plunger component comprises a plunger attached to a handle, wherein the plunger is configured slideable along an inner side of the inner wall of the liquid reservoir.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

### LIST OF REFERENCE SIGNS

- 10, 10a: electronic smoking device
- 12: power supply portion
- 14: atomizer/liquid reservoir portion
- 16: end cap
- 18: battery
- 20: light-emitting diode (LED)
- 22: control electronics
- 24: airflow sensor
- 26: atomizer
- 28: heating coil
- 30: wick
- 32: central passage
- 33, 133: outer refill interface
- 33-1, 33-2, 133-1, 133-2: separate opening
- 34: liquid reservoir
- 36: air inhalation port
- 38: air inlets
- 39: mouthpiece
- 100, 1100: liquid reservoir of syringe refiller
- 100a, 1100a: first chamber of the liquid reservoir
- 100b, 1100b: second chamber of the liquid reservoir
- 101, 1101: first end
- 102, 1102: second end
- 105, 1105: inner wall
- 110, 1110: first opening
- 120, 1120: second opening
- 121: third opening
- 130, 1130: plunger component
- 131, 1131: rod shaped shaft
- 132, 1132: inlay gasket
- 135, 1135: hollow body
- 135-1: first end of the hollow body
- 135-2: second end of the hollow body
- 140, 1140: plunger
- 140-1: peripheral cavity
- 141, 1141: outer gasket element
- 145: hole
- 145-1: center area
- 150, 1150: handle
- 151: stopper body
- 160, 1160: first syringe needle element
- 169, 1169: air channelling system
- 170, 1170: air channel component
- 170-1, 1170-1: first channel opening
- 170-2, 1170-2: second channel opening
- 171: internal gasket element
- 172: fraction
- 180: second syringe needle element
- 182: child-safety cap
- 182-1: first hole in the child-safety cap
- 182-2: second hole in the child-safety cap
- 183: cylindrical base part
- 183-1: groove-like depressions
- 184: nozzle part
- 184-1: nozzle cavity
- 190: stopper element
- 190-1: first cylindrical portion of the stopper element
- 190-2: second cylindrical portion of the stopper element
- 190-3: third cylindrical portion of the stopper element
- 191: reception cavity
- 192: spring element
- 193: releasable connection element
- 194: first corresponding engagement element
- 195: inlay collar
- 195-1: first cylindrical portion of the inlay collar
- 195-2: second cylindrical portion of the inlay collar
- 196: second corresponding engagement element
- 200, 1200: syringe refiller
- CL: center line
- La: line

## Claims

1. A syringe refiller (200) for an electronic smoking device (10), comprising
a liquid reservoir (100) comprising a first end (101) with a first opening (110) for the reception of a plunger component (130) and a second end (102) with a second opening (120) for a liquid to be pushed out of the liquid reservoir (100); and
a plunger component (130) comprising a plunger (140) attached to a handle (150), the plunger (140) being configured slidable along an inner wall (105) of the liquid reservoir (100), wherein the plunger (140) seals the liquid reservoir (100) towards the first opening (110), and wherein the plunger component (130) comprises a hollow body (135) with a first end (135-1) attached to the plunger (140) and a second end (135-2) attached to the handle (150), the plunger (140) comprising a hole (145) for the insertion of an air channel component (170) of an air channelling system (169);
a first syringe needle element (160) that is in connection with the second opening (120), so that liquid which is pushed out of the second opening (120) via the plunger component (130) flows through the syringe needle element (160), and
an air channelling system (169),
**characterized in that**,
the air channelling system (169) comprises an air channel component (170) inserted into the hole (145) of the plunger (145), adapted to transport air from outside of the syringe refiller (200) into the hollow body (135), allowing for air to be sucked into the syringe refiller (200) without interfering with liquid contained therein, wherein the plunger (140) is configured slidable along the air channel component (170), wherein said air channel component (170) comprises a first channel opening (170-1) and at least one second channel opening (170-2), the first channel opening (170) being arranged within the hollow body (135) and the at least one second channel opening (170-2) being arranged adjacent to the second opening (120) within a sidewall at the second end (102) of the liquid reservoir (100).

2. The syringe refiller (200) of claim 1, wherein the air channelling system (169) comprises an air channel component (170) and an internal gasket element (171), the internal gasket element (171) being arranged within the hole (145) of the plunger (140), providing for an airtight contact between the plunger (140) and the air channel component (170).

3. The syringe refiller (200) of claim 2, wherein the second channel opening (170-2) is connected to a second syringe needle element (180), adapted for the suction of air.

4. The syringe refiller (200) of any one of the previous claims, further comprising a stopper element (190) arranged at the second end (102) with the second opening (120) arranged within the stopper element (190) and an inlay collar (195) arranged at the first end (101) of the liquid reservoir (100), wherein the first opening (110) is arranged within the inlay collar (195).

5. The syringe refiller (200) of claims 3 and 4, wherein the first and the second syringe needle elements (160, 180) are arranged within the stopper element (190) and protrude from the stopper element (190) along a direction that is pointing away from the liquid reservoir (100) respectively.

6. The syringe refiller (200) of claim 5, wherein the stopper element (190) further comprises a reception cavity (191) that receives the fraction (172) of the air channel component (170) that comprises the second channel opening (170-2).

7. The syringe refiller (200) of any one of the previous claims, further comprising a child-safety cap (182) arranged at the second end (102) of the liquid reservoir (100), being adapted to fully enclose the first syringe needle element (160) in a locked state of the syringe refiller (200) and to release the first syringe needle element (160) in an unlocked state of the syringe refiller (200).

8. The syringe refiller (200) of claim 7 and any one of the claims 4 to 6, wherein the stopper element (190) comprises a spring element (192), wherein the child-safety cap (182) is configured slidable along the stopper element (190) and wherein the child-safety cap (182) is interacting with the spring element (192) when slid along the stopper element (190) .

9. The syringe refiller (200) of claim 8, wherein the child-safety cap (182) further comprises a first hole (182-1) arranged such that the first syringe needle element (160) pierces through the child-safety cap (182) when the child-safety cap (182) is in the unlocked state of the syringe refiller (200).

10. The syringe refiller (200) of claim 8 or 9, wherein the stopper element (190) comprises at least one releasable connection element (193) on at least one outer side of the stopper element (190), the releasable connection element (193) being adapted to engage with a first corresponding engagement element (194) arranged within the child-safety cap (182) in an unlocked state of the syringe refiller (200).

11. The syringe refiller (200) of claim 10, wherein the releasable connection element (193) is a retaining lug and the first corresponding engagement element (194) is a fixation hole, the retaining lug and the fixation hole together form a snap-action connection.

12. The syringe refiller (200) of claim 10 or 11, wherein a second corresponding engagement element (196) is arranged within the child-safety cap (182), adapted to engage with the at least one releasable connection element (193) in a locked state of the syringe refiller (200).

13. The syringe refiller (200) of any one of the claims 7 to 12 and claim 3, wherein the child-safety cap (182) is further adapted to fully enclose the second syringe needle element (180) in a locked state and to release the second syringe needle element (180) in an unlocked state.

14. The syringe refiller (200) of claim 13, wherein the child-safety cap (182) further comprises a second hole (182-2) arranged such that the second syringe needle element (180) pierces through the child-safety cap (182) when the syringe refiller (200) is in the unlocked state.

15. An electronic smoking device system (300), comprising
an electronic smoking device (10) with at least one refill opening (33-1, 33-2);
a syringe refiller (200) of any one of the claims 1 to 14.

## Patentansprüche

1. Spritzennachfüller (200) für eine elektronische Rauchvorrichtung (10), umfassend:
einen Flüssigkeitsbehälter (100), der ein erstes Ende (101) mit einer ersten Öffnung (110) zur Aufnahme einer Kolbenkomponente (130) und ein zweites Ende (102) mit einer zweiten Öffnung (120) für eine aus dem Flüssigkeitsbehälter (100) herauszudrückende Flüssigkeit, umfasst; und
eine Kolbenkomponente (130), die einen Kolben (140) umfasst, der an einem Griff (150) befestigt ist, wobei der Kolben (140) dazu ausgestaltet ist, schiebbar entlang einer inneren Wand (105) des Flüssigkeitsbehälters (100) zu sein, wobei der Kolben (140) den Flüssigkeitsbehälter (100) zu der ersten Öffnung (110) hin abdichtet, und wobei die Kolbenkomponente (130) einen Hohlkörper (135) mit einem an dem Kolben (140) befestigten ersten Ende (135-1) und einem an dem Griff (150) befestigten zweiten Ende (135-2) umfasst, wobei der Kolben (140) ein Loch (145) zum Einsetzen einer Luftkanalkomponente (170) eines Luftkanalsystems (169) umfasst;
ein erstes Spritzennadelelement (160), das in Verbindung mit der zweiten Öffnung (120) steht, sodass Flüssigkeit, die aus der zweiten Öffnung (120) über die Kolbenkomponente (130) herausgedrückt wird, durch das Spritzennadelelement (160) fließt, und
ein Luftkanalsystem (169),
**dadurch gekennzeichnet, dass**
das Luftkanalsystem (169) eine Luftkanalkomponente (170) umfasst, die in das Loch (145) des Kolbens (145) eingesetzt ist, die dazu ausgelegt ist, Luft von außerhalb des Spritzennachfüllers (200) in den Hohlkörper (135) zu transportieren, wodurch ermöglicht wird, dass Luft in den Spritzennachfüller (200) hineingesogen wird, ohne die darin enthaltene Flüssigkeit zu behindern, wobei der Kolben (140) schiebbar entlang der Luftkanalkomponente (170) ausgestaltet ist, wobei die Luftkanalkomponente (170) eine erste Kanalöffnung (170-1) und mindestens eine zweite Kanalöffnung (170-2) umfasst, wobei die erste Kanalöffnung (170) innerhalb des Hohlkörpers (135) angeordnet ist und die mindestens eine zweite Kanalöffnung (170-2) benachbart zu der zweiten Öffnung (120) innerhalb einer Seitenwand an dem zweiten Ende (102) des Flüssigkeitsbehälters (100) angeordnet ist.

2. Spritzennachfüller (200) nach Anspruch 1, wobei das Luftkanalsystem (169) eine Luftkanalkomponente (170) und ein inneres Dichtungselement (171) umfasst, wobei das innere Dichtungselement (171) innerhalb des Loches (145) des Kolbens (140) angeordnet ist, wodurch ein luftdichter Kontakt zwischen dem Kolben (140) und der Luftkanalkomponente (170) bereitgestellt wird.

3. Spritzennachfüller (200) nach Anspruch 2, wobei die zweite Kanalöffnung (170-2) mit einem zweiten Spritzennadelelement (180) verbunden ist, das zum Ansaugen von Luft ausgelegt ist.

4. Spritzennachfüller (200) nach einem der vorangehenden Ansprüche, ferner umfassend ein Verschlusselement (190), das an dem zweiten Ende (102) angeordnet ist, wobei die zweite Öffnung (120) innerhalb des Verschlusselementes (190) angeordnet ist, und einen an dem ersten Ende (101) des Flüssigkeitsbehälters (100) angeordneter Einfassungsring (195), wobei die erste Öffnung (110) innerhalb des Einfassungsrings (195) angeordnet ist.

5. Spritzennachfüller (200) nach Anspruch 3 und 4, wobei das erste und das zweite Spritzennadelelement (160, 180) innerhalb des Verschlusselements (190) angeordnet sind und aus dem Verschlusselement (190) entlang einer Richtung hervorragen, die jeweils von dem Flüssigkeitsbehälter (100) weg zeigt.

6. Spritzennachfüller (200) nach Anspruch 5, wobei das Verschlusselement (190) ferner eine Aufnahmevertiefung (191) umfasst, die den Teil (172) der Luftkanalkomponente (170) aufnimmt, der die zweite Kanalöffnung (170-2) umfasst.

7. Spritzennachfüller (200) nach einem der vorangehenden Ansprüche, ferner umfassend eine Kindersicherungskappe (182), die an dem zweiten Ende (102) des Flüssigkeitsbehälters (100) angeordnet ist, die dazu ausgelegt ist, das erste Spritzennadelelement (160) in einem verriegelten Zustand des Spritzennachfüllers (200) vollständig zu umgeben und das erste Spritzennadelelement (160) in einem entriegelten Zustand des Spritzennachfüllers (200) zu lösen.

8. Spritzennachfüller (200) nach Anspruch 7 und einem der Ansprüche 4 bis 6, wobei das Verschlusselement (190) ein Federelement (192) umfasst, wobei die Kindersicherungskappe (182) schiebbar entlang des Verschlusselementes (190) ausgestaltet ist und wobei die Kindersicherungskappe (182) mit dem Federelement (192) in Wechselwirkung steht, wenn sie entlang dem Verschlusselement (190) geschoben wird.

9. Spritzennachfüller (200) nach Anspruch 8, wobei die Kindersicherungskappe (182) ferner ein erstes Loch (182-1) umfasst, das so angeordnet ist, dass das erste Spritzennadelelement (160) durch die Kindersicherungskappe (182) durchsticht, wenn die Kindersicherungskappe (182) sich in dem entriegelten Zustand des Spritzennachfüllers (200) befindet.

10. Spritzennachfüller (200) nach Anspruch 8 oder 9, wobei das Verschlusselement (190) mindestens ein lösbares Verbindungselement (193) auf mindestens einer Außenseite des Verschlusselementes (190) umfasst, wobei das lösbare Verbindungselement (193) dazu ausgelegt ist, in ein erstes entsprechendes Eingriffselement (194) einzugreifen, das innerhalb der Kindersicherungskappe (182) in einem entriegelten Zustand des Spritzennachfüllers (200) angeordnet ist.

11. Spritzennachfüller (200) nach Anspruch 10, wobei das lösbare Verbindungselement (193) eine Haltenase ist und das erste entsprechende Eingriffselement (194) ein Befestigungsloch ist, wobei die Haltenase und das Befestigungsloch gemeinsam eine Schnappwirkverbindung ausbilden.

12. Spritzennachfüller (200) nach Anspruch 10 oder 11, wobei ein zweites entsprechendes Eingriffselement (196), das innerhalb der Kindersicherungskappe (182) angeordnet ist, dazu ausgelegt ist, in das mindestens eine lösbare Verbindungselement (193) in einem verriegelten Zustand des Spritzennachfüllers (200) einzugreifen.

13. Spritzennachfüller (200) nach einem der Ansprüche 7 bis 12 und Anspruch 3, wobei die Kindersicherungskappe (182) ferner dazu ausgelegt ist, das zweite Spritzennadelelement (180) in einem verriegelten Zustand vollständig zu umgeben und das zweite Spritzennadelelement (180) in einem entriegelten Zustand zu lösen.

14. Spritzennachfüller (200) nach Anspruch 13, wobei die Kindersicherungskappe (182) ferner ein zweites Loch (182-2) umfasst, das so angeordnet ist, dass das zweite Spritzennadelelement (180) die Kindersicherungskappe (182) durchsticht, wenn der Spritzennachfüller (200) sich in dem entriegelten Zustand befindet.

15. Elektronisches Rauchvorrichtungssystem (300), umfassend
eine elektronische Rauchvorrichtung (10) mit mindestens einer Nachfüllöffnung (33-1, 33-2);
einen Spritzennachfüller (200) nach einem der Ansprüche 1 bis 14.

## Revendications

1. Dispositif de rechargement de seringue (200) pour un dispositif de cigarette électronique (10), comprenant
un réservoir de liquide (100) comprenant une première extrémité (101) munie d'une première ouverture (110) pour la réception d'un composant de piston (130) et une deuxième extrémité (102) munie d'une deuxième ouverture (120) pour un liquide à pousser hors du réservoir de liquide (100) ; et
un composant de piston (130) comprenant un piston (140) attaché à une poignée (150), le piston (140) étant conçu pour pouvoir coulisser le long d'une paroi intérieure (105) du réservoir de liquide (100), le piston (140) fermant hermétiquement le réservoir de liquide (100) vers la première ouverture (110), et le composant de piston (130) comprenant un corps creux (135) muni d'une première extrémité (135-1) attachée au piston (140) et d'une deuxième extrémité (135-2) attachée à la poignée (150), le piston (140) comprenant un trou (145) pour l'insertion d'un composant de canal d'air (170) d'un système de canalisation d'air (169) ;
un premier élément d'aiguille de seringue (160) qui est en connexion avec la deuxième ouverture (120), de telle sorte que le liquide qui est poussé hors de la deuxième ouverture (120) via le composant de piston (130) s'écoule à travers l'élément d'aiguille de seringue (160), et
un système de canalisation d'air (169),
**caractérisé en ce que**
le système de canalisation d'air (169) comprend un composant de canal d'air (170) inséré dans le trou (145) du piston (130), adapté pour transporter de l'air de l'extérieur du dispositif de rechargement de seringue (200) dans le corps creux (135), permettant à de l'air d'être aspiré dans le dispositif de rechargement de seringue (200) sans interférer avec le liquide contenu dans celui-ci, le piston (140) étant conçu pour pouvoir coulisser le long du composant de canal d'air (170), ledit composant de canal d'air (170) comprenant une première ouverture de canal (170-1) et au moins une deuxième ouverture de canal (170-2), la première ouverture de canal (170) étant agencée à l'intérieur du corps creux (135) et l'au moins une deuxième ouverture de canal (170-2) étant agencée de manière adjacente à la deuxième ouverture (120) à l'intérieur d'une paroi latérale au niveau de la deuxième extrémité (102) du réservoir de liquide (100).

2. Dispositif de rechargement de seringue (200) selon la revendication 1, le système de canalisation d'air (169) comprenant un composant de canal d'air (170) et un élément de joint interne (171), l'élément de joint interne (171) étant agencé à l'intérieur du trou (145) du piston (140), fournissant un contact étanche à l'air entre le piston (140) et le composant de canal d'air (170).

3. Dispositif de rechargement de seringue (200) selon la revendication 2, la deuxième ouverture de canal (170-2) étant connectée à un deuxième élément d'aiguille de seringue (180), adapté pour l'aspiration d'air.

4. Dispositif de rechargement de seringue (200) selon l'une quelconque des revendications précédentes, comprenant en outre un élément de bouchon (190) agencé à la deuxième extrémité (102) avec la deuxième ouverture (120) agencée à l'intérieur de l'élément de bouchon (190) et un collet d'insertion (195) agencé à la première extrémité (101) du réservoir de liquide (100), la première ouverture (110) étant agencée à l'intérieur du collet d'insertion (195).

5. Dispositif de rechargement de seringue (200) selon les revendications 3 et 4, le premier et le deuxième élément d'aiguille de seringue (160, 180) étant agencés à l'intérieur de l'élément de bouchon (190) et faisant saillie de l'élément de bouchon (190) le long d'une direction qui est dirigée respectivement à l'opposé du réservoir de liquide (100).

6. Dispositif de rechargement de seringue (200) selon la revendication 5, l'élément de bouchon (190) comprenant en outre une cavité de réception (191) qui reçoit la fraction (172) du composant de canal d'air (170) qui comprend la deuxième ouverture de canal (170-2).

7. Dispositif de rechargement de seringue (200) selon l'une quelconque des revendications précédentes, comprenant en outre un capuchon de sécurité enfant (182) agencé à la deuxième extrémité (102) du réservoir de liquide (100), étant adapté pour enfermer complètement le premier élément d'aiguille de seringue (160) dans un état verrouillé du dispositif de rechargement de seringue (200) et pour libérer le premier élément d'aiguille de seringue (160) dans un état déverrouillé du dispositif de rechargement de seringue (200).

8. Dispositif de rechargement de seringue (200) selon la revendication 7 et l'une quelconque des revendications 4 à 6, l'élément de bouchon (190) comprenant un élément de ressort (192), le capuchon de sécurité enfant (182) étant conçu pour pouvoir coulisser le long de l'élément de bouchon (190), et le capuchon de sécurité enfant (182) interagissant avec l'élément de ressort (192) lorsqu'il coulisse le long de l'élément de bouchon (190).

9. Dispositif de rechargement de seringue (200) selon la revendication 8, le capuchon de sécurité enfant (182) comprenant en outre un premier trou (182-1) agencé de telle sorte que le premier élément d'aiguille de seringue (160) perce à travers le capuchon de sécurité enfant (182) lorsque le capuchon de sécurité enfant (182) est dans l'état déverrouillé du dispositif de rechargement de seringue (200).

10. Dispositif de rechargement de seringue (200) selon la revendication 8 ou 9, l'élément de bouchon (190) comprenant au moins un élément de connexion libérable (193) sur au moins un côté extérieur de l'élément de bouchon (190), l'élément de connexion libérable (193) étant adapté pour s'engager avec un premier élément d'engagement correspondant (194) agencé à l'intérieur du capuchon de sécurité enfant (182) dans un état déverrouillé du dispositif de rechargement de seringue (200).

11. Dispositif de rechargement de seringue (200) selon la revendication 10, l'élément de connexion libérable (193) étant une patte de retenue et le premier élément d'engagement correspondant (194) étant un trou de fixation, la patte de retenue et le trou de fixation formant ensemble une connexion par encliquetage.

12. Dispositif de rechargement de seringue (200) selon la revendication 10 ou 11, un deuxième élément d'engagement correspondant (196) étant agencé à l'intérieur du capuchon de sécurité enfant (182), adapté pour s'engager avec l'au moins un élément de connexion libérable (193) dans un état verrouillé du dispositif de rechargement de seringue (200).

13. Dispositif de rechargement de seringue (200) selon l'une quelconque des revendications 7 à 12 et la revendication 3, le capuchon de sécurité enfant (182) étant en outre adapté pour enfermer complètement le deuxième élément d'aiguille de seringue (180) dans un état verrouillé et pour libérer le deuxième élément d'aiguille de seringue (180) dans un état déverrouillé.

14. Dispositif de rechargement de seringue (200) selon la revendication 13, le capuchon de sécurité enfant (182) comprenant en outre un deuxième trou (182-2) agencé de telle sorte que le deuxième élément d'aiguille de seringue (180) perce à travers le capuchon de sécurité enfant (182) lorsque le dispositif de rechargement de seringue (200) est dans l'état déverrouillé.

15. Système de dispositif de cigarette électronique (300), comprenant :
un dispositif de cigarette électronique (10) muni d'au moins une ouverture de rechargement (33-1, 33-2) ;
un dispositif de rechargement de seringue (200) selon l'une quelconque des revendications 1 à 14.
